(19) Europäisches Patentamt
European Patent Office
Office européen des brevets

(11) EP 2 789 690 A1

(12) EUROPEAN PATENT APPLICATION
published in accordance with Art. 153(4) EPC

(43) Date of publication:
**15.10.2014 Bulletin 2014/42**

(21) Application number: **12855465.6**

(22) Date of filing: **14.11.2012**

(51) Int Cl.:
***C12N 15/63*** *(2006.01)* ***C12N 15/52*** *(2006.01)*
***C12N 9/00*** *(2006.01)* ***A01H 1/00*** *(2006.01)*
***A01H 5/00*** *(2006.01)*

(86) International application number:
**PCT/CN2012/001546**

(87) International publication number:
**WO 2013/082865 (13.06.2013 Gazette 2013/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.12.2011 CN 201110406266**

(71) Applicant: **Institute of Botany of The Chinese Academy of Sciences**
**Beijing 100093 (CN)**

(72) Inventors:
- **QI, Xiaoquan**
  **Beijing 100093 (CN)**
- **XUE, Zheyong**
  **Beijing 100093 (CN)**
- **ZHANG, Yingchun**
  **Beijing 100093 (CN)**
- **XU, Xia**
  **Beijing 100093 (CN)**
- **LIU, Dan**
  **Beijing 100093 (CN)**

(74) Representative: **Fleck, Barbara**
**Marks & Clerk LLP**
**62-68 Hills Road**
**Cambridge CB2 1LA (GB)**

# (54) METHOD FOR PREPARING FERTILITY-LOWERED PLANT

(57) The present invention relates to a method for preparing fertility-lowered plant. The present invention provides an RNA interference vector and a method for obtaining a transgenic plant by introducing said RNA interference vector into a target plant; said transgenic plant is the following 1) or 2): 1) Sterile transgenic plant or 2) The fertility of said transgenic plant is lower than said target plant; the present invention also provides a method for cultivating a target plant to a sterile mutant or fertility-lowered mutant by sodium azide mutagenesis. The experiments of the present invention proved that the present invention provides various methods for preparing sterile lines or fertility-lowered lines; including RNA interference or TILLING (Targeting Induced Local Lesions IN Genomes) technology selection; sterile lines prepared by the methods of the present invention establish the basis of rice heterosis and crossbreeding.



Printed by Jouve, 75001 PARIS (FR)

## Description

### TECHNICAL FIELD

The present invention relates to biotechnology, especially to a method for preparing a fertility-lowered plant.

### BACKGROUND

Male sterility in plant is a botanical characteristic closely related to agricultural production and is the result of interaction between gene expression and environment during plant development. Autologous male sterility in plant can be used in breeding research including development and utilization of crop heterosis, conducting recurrent selection and backcross, etc. as a genetic tool without artificial emasculation. It provides the possibility to produce a large amount of hybrid seeds by utilizing plant male sterility to breed various male sterile lines and then producing hybrid seeds in large quantities by means of genetic engineering so that heterosis of many crops especially self-pollinated crops can be utilized in production.

Rice is an important cereal crop in China. The discoveries of rice heterosis and hybrid breeding methods make great contribution to rice production, increasing the production by more than 20% compared with conventional breeding. Following the discovery of Enviroment-sensitive Genetic Male-sterile Rice, national academy member YUAN Long-ping put forward a two-line breeding method which makes the strategy of rice hybrid breeding simple and convenient, improves the quality in many aspects and increases the production by 5-10%. Nongken 58S is the first photoperiod-sensitive male sterile line which as well as its transformed japonica rice sterile line and indica rice sterile line can be induced to be sterile by long day and fertile by short day. Meanwhile, they are also called Photo thermo sensitive genetic male sterile line because their fertility is affected by temperature. By means of gene mapping, it is found that the Photo thermo sensitive genetic male sterility is regulated by a noncoding precursor RNA. However, abnormal low temperature (<23 °C) in the summer will lead to failure in production of hybrid seeds, so the use of photo thermo sensitive genetic male sterile lines are affected to some extent.

Besides the influence of light and temperature, humidity is also an important environment factor relating to plant growth and development. To date, there isn't any report on the relationship between humidity and rice male sterility. However, a male sterile mutant *pop1* which is related to humidity was found in *Arabidopsis thaliana* as early as 1993. The pollen of the mutant cannot absorb water from the stigma due to the lack of long chain fatty acid and wax in the tryphine of the pollen surface, thereby leading to the failure of pollination and sterility (but the fertility of the mutant was restored by transferring the mutant from environment with a relative humidity of 70% to a high humidity box with a relative humidity of 90%. Preuss et al., Gene Dev. 1993, 7:947-985).

### SUMMARY

In one aspect, the present invention provides an RNA interference vector.

The RNA interference vector of the invention is obtained by inserting the DNA molecule as shown in SEQ ID NO.1 into a pH7GWIWG2(II) vector.

The above RNA interference vector is obtained by inserting the DNA molecule as shown in SEQ ID NO.1 into a pH7GWIWG2(II) vector by means of homologous recombination. In particular, the vector is obtained by inserting the DNA molecule as shown in SEQ ID NO. 1 into a pH7GWIWG2(II) vector in a forward direction and reverse direction by means of homologous recombination.

The above RNA interference vector is prepared according to a method comprising the following steps:

1) Obtaining an intermediate vector with the DNA molecule as shown in SEQ ID NO.1 and a pDONR221 vector by BP reaction;
2) Obtaining an RNA interference vector with said intermediate vector and a pH7GWIWG2(II) vector by LR reaction.

The aforementioned DNA molecule as shown in SEQ ID NO.1 is prepared according to the following method: PCR amplification of rice cDNAs with primer pair A, and the obtained PCR product is the DNA molecule as shown in SEQ ID NO.1.

Said primer pair A consists of the single chain DNAs as shown in SEQ ID NO.3 and SEQ ID NO.4.

Recombinant bacteria or a transgenic cell line comprising said RNA interference vector also fall into the protection scope of the present invention.

Use of said RNA interference vector, recombinant bacteria or transgenic cell line in cultivating rice sterile lines or reducing rice fertility also falls in the protection scope of the present invention.

The second aspect of the present invention is to provide a method for cultivating a transgenic plant.

The method provided by the present invention is directed to obtain the transgenic plant by introducing said RNA interference vector into a target plant; wherein said transgenic plant comprises the following 1) or 2):

1) Sterile transgenic plant; or 2) The fertility of said transgenic plant is lower than said target plant; wherein said target plant is specifically a monocot plant; said monocot plant is specifically rice.

The transgenic plant obtained by said method is also within the protection scope of the present invention; Said transgenic plant is a sterile transgenic plant or fertility-lowered transgenic plant; wherein said plant is specifically a monocot plant; said monocot plant is further specifically rice. Said fertility-lowered transgenic plant is a transgenic plant whose fertility is lower than the target plant.

The third aspect of the present invention is directed to provide a method for cultivating a target plant into a sterile mutant or fertility-lowered mutant.

The method provided by the present invention comprises the following steps:

1) Mutating seeds of a target plant; Using primer pair B and fluorescently labeled primer pair B to specifically amplify genes encoding triterpene synthase from a target plant;
wherein mutating the seeds of a target plant is performed by treating a number of seeds of the target plant with sodium azide, thereby obtaining the mutated seeds;
wherein said primer pair B is used to specifically amplify the triterpene synthase in the target plant is designed according to the particular sequences of triterpene synthase gene in the target plant; wherein each primer of said fluorescently labeled primer pair B is labeled with different fluorescently labeled probes; wherein said different fluorescently labeled probes have different wavelengths;
2) Cultivating said mutagenized seeds to obtain the first generation of mutation M1; Self-cross of the first generation of mutation M1 was carried out to obtain the second generation of mutation M2;
3) Extracting genomic DNA of individual plant of the second generation of mutation M2; Mixing genomic DNA of individual plants of M2 (with number n) to obtain a DNA pool; wherein n is 2-8;
4) Using each said DNA pool as a template and both said primer pair B and fluorescently labeled primer pair B to perform amplification, so as to obtain PCR products;
5) Digesting said PCR products with endonuclease CELI to obtain enzyme-digested products of the DNA pool;
6) detecting the enzyme-digested products of each said DNA pool by electrophoresis; wherein if enzyme-digested products of said DNA pool generate bright dots under all wavelengths of different fluorescently labeled probes, individual plants of M2 generation (with number n) represented by said DNA pool contain or may contain fertility-lowered mutants or sterile mutants; wherein if the enzyme-digested products of said DNA pool do not generate bright dots under all wavelengths of different fluorescently labeled probes, M2 generation (number n) represented by said DNA pool do not contain or may not contain fertility-lowered mutants or sterile mutants; wherein said fertility-lowered mutants are plants whose fertility is lower than that of said target plant.

Following said step 6), said method further comprises the following steps:

Genomic DNA of individual plant of M2 (with number n) which contain or may contain fertility-lowered mutants or sterile mutants is mixed with genomic DNA of said target plant; steps 4)-5) are repeated to obtain enzyme-digested products of individual plant of M2.

Each of said enzyme-digested products of individual plant of M2 is detected by electrophoresis; wherein if said enzyme-digested products of individual plant of M2 generate bright dots under all wavelengths of different fluorescently labeled probes, said individual plant of M2 is or may be a sterile mutant or fertility-lowered mutant; wherein if said enzyme-digested products of individual plant of M2 do not generate bright dot under all wavelengths of different fluorescently labeled probes, said individual plant of M2 is not or may not be a sterile mutant or fertility-lowered mutant.

In step 1) of said method, treating a number of seeds of the target plant with sodium azide comprises immersing a number of seeds of the target plant in an aqueous solution of sodium azide with a concentration of 2mM for 6 hours under room temperature.

The amino acid sequence of said triterpene synthase is shown in SEQ ID NO. 2;

Wherein said different fluorescently labeled probes refer to fluorescently labeled probe DY-682 with a wavelength of 682nm and fluorescently labeled probe DY-782 with a wavelength of 782nm;

The nucleotide sequence of the gene encoding said triterpene synthase is shown in SEQ ID NO. 5;

The primer pair B is one of the following primer pairs represented by 1)-3):

1) Primer pair consisting of the single chain DNA molecule as shown in SEQ ID NO. 6 and the single chain DNA molecule as shown in SEQ ID NO. 7;
2) Primer pair consisting of the single chain DNA molecule as shown in SEQ ID NO. 8 and the single chain DNA molecule as shown in SEQ ID NO. 9;
3) Primer pair consisting of the single chain DNA molecule as shown in SEQ ID NO. 10 and the single chain DNA molecule as shown in SEQ ID NO. 11;

wherein said target plant is a monocot plant; wherein said monocot plant is a monocot graminaceous plant; specifically, said monocot graminaceous plant is rice.

The above fertility-lowered mutant is a mutant whose fertility is lower than the target plant. In one embodiment, the target plant is specifically rice, and fertility-lowered mutant is a mutant whose fertility is lower than that of taget rice.

Sterile mutant or fertility-lowered mutants prepared by the above method are also within the protection scope of the present invention.

The Deposit Number of the above-mentioned fertility-lowered mutant is CGMCC NO. 6150.

Use of the above transgenic plants or the above sterile mutant or fertility-lowered mutant in the production of hybrid seeds is also within the scope of protection of the present invention.

The above fertility-lowered mutant is a mutant whose fertility is lower than that of a target plant; said target plant is a monocot plant; said monocot plant is a monocot graminaceous plant; said monocot graminaceous plant is specifically rice; In one embodiment, a fertility-lowered mutant is specifically a mutant whose fertility is lower than that of target rice.

The fourth aspect of the present invention is to provide a method for obtaining a sterile mutant or fertility-lowered mutant.

The method provided by the present invention is to obtain a sterile mutant or fertility-lowered mutant by silencing or inactivating the gene encoding triterpene synthase in a target plant; said fertility-lowered mutant is a plant whose fertility is lower than that of the target plant.

In the above method, said target plant is a monocot or dicot plant; said monocot plant is specifically a monocot graminaceous plant;
said monocot graminaceous plant is rice, wheat, barley, sorghum or maize;

The amino acid sequence of the triterpene synthase of rice is shown in SEQ ID NO. 2; the nucleotide sequence of the gene encoding triterpene synthase of rice is shown in SEQ ID NO. 5.

The amino acid sequence of the triterpene synthase of wheat is shown in SEQ ID NO. 15; the nucleotide sequence of the gene encoding triterpene synthase of wheat is shown in SEQ ID NO. 14.

The amino acid sequence of the triterpene synthase of barley is shown in SEQ ID NO. 17; the nucleotide sequence of the gene encoding triterpene synthase of barley is shown in SEQ ID NO. 16.

The amino acid sequence of the triterpene synthase of sorghum is shown in SEQ ID NO. 18; the nucleotide sequence of the gene encoding triterpene synthase of sorghum is shown in SEQ ID NO. 19.

The amino acid sequence of the triterpene synthase of maize is shown in SEQ ID NO. 20; the nucleotide sequence of the gene encoding triterpene synthase of maize is shown in SEQ ID NO. 21.

The method of the aforementioned silencing or inactivation of the gene encoding triterpene synthase in a target plant can be specifically RNA interference of expression of the gene encoding triterpene synthase in a target plant or point mutation of the gene encoding triterpene synthase in a target plant.

In the above method, said silencing or inactivating of the gene encoding triterpene synthase in rice can be at least one of the following 1)-3):

1) The nucleotide residue at the 764 position of the gene encoding triterpene synthase in rice is mutated from G to A;
2) The nucleotide residue at the 809 position of the gene encoding triterpene synthase in rice is mutated from G to A;
3) The nucleotide residue at the 1431 position of the gene encoding triterpene synthase in rice is mutated from G to A.

The fifth aspect of the present invention is to provide a method for restoring or improving fertility of an original plant.

The method provided by the present invention comprises the following steps: maintaining the humidity for growth of plant inflorescence at 80-100% during anthesis of an original plant; wherein said original plant is a sterile mutant or fertility-lowered mutant.

In the above method, said sterile mutant or fertility-lowered mutant is the aforementioned transgenic plant or the aforementioned sterile mutant or fertility-lowered mutant.

In the above method, the time period of said maintaining the humidity for growth of plant inflorescence is one week;
the method for maintaining the humidity for growth of plant inflorescence comprises wrapping the whole inflorescence of said original plant;

said wrapping comprises specifically using a plastic bag to slip over the whole inflorescence or using preservative film to cover the whole inflorescence.

The fertility-lowered mutant named P34E8 selected above is a mutant strain of OsOSC8 (mutant S6) which has been deposited in China General Microbiological Culture Collection Center (CGMCC for short, address: No.3, Courtyard No. 1, West Road Beichen, Chaoyang District, Beijing) on 28 May 2012 with a deposit Number of CGMCC No. 6150; the classification and nomenclature is rice (*Oryza sativa*).

Unless particularly noted or separately defined, the terms of science and technology used in this text have the unambiguously same meaning as is well known to the person skilled in the art. Moreover, the material, method and enbodiments of the text are intended to explain and elaborate the present invention rather than limitation or restriction.

## DESCRIPTION OF DRAWINGS

Figure 1 shows result of western blot analysis of the protein of RNAi lines.

Figure 2 shows statistical result of setting percentage under natural condition and setting percentage after moisturizing treatment.

Figure 3 shows electrophoretogram of detected mutants.

Figure 4 shows inflorescence (A, B), floret (C, D, bars=0.5cm), $I_2$-KI staining (E, F, bars=100$\mu$m) and Alexander staining (G, H, bars=100$\mu$m) of wild type (WT) and mutant (P34E8).

Figure 5 shows germination of pollens of wild type (WT) and mutant (P34E8) in culture medium, bars=100$\mu$m.

Figure 6 shows adhesion and germination of pollens of wild type (WT) and mutant (P34E8) on stigmas at different times, bars=100$\mu$m.

Figure 7 shows adhesion and germination of pollens on stigmas after reciprocal cross is made between wild type (WT) and mutant (P34E8), bars=100$\mu$m.

Figure 8 shows fructification of inflorescence of wild type (WT), mutant (P34E8) and homozygous mutant after moisturizing treatment, bars=2cm.

Figure 9 shows electrophoretogram of amplified fragments of homologous genes in barley and wheat.

Figure 10 shows sequence alignment of homologous proteins of OsOSC8 in graminaceous crops and possible effective mutant sites.

## EMBODIMENTS

Unless specially illustrated, the experimental methods used in the following embodiments are all conventional methods.

Unless specially illustrated, all of the materials and reagents, etc. used in the following embodiments can be obtained from commercial sources.

All of the quantitative tests in the following embodiments are repeated three times, and the results are mean value or mean value$\pm$standard deviation.

The amino acid sequence of triterpene synthase OsOSC8 is shown in SEQ ID NO.2; the nucleotide sequence encoding the gene is shown in SEQ ID NO.5.

Example 1: Preparation of fertility-lowered transgenic rice by RNA interference

I Obtaining of RNA interference vector of OsOSC8

1. Obtaining of Gateway intermediate vector pDONR221/osc8-1.

The primers were designed according to the gene sequence of OsOSC8: Sequence attB1 was added to 5' end of the sense primer and sequence attB2 was added to 5' end of the antisense primer by using Gateway technology of Invitrogen, USA.

Primer pair 1: sense: 5'-AAAAAGCAGGCTGGCTGCACGGATAGAGTT-3' (SEQ ID NO.3)

antisense: 5'-AGAAAGCTGGGTGCCTGTATGGCTGAGAAA-3' (SEQ ID NO.4)

Total RNA was extracted from rice Zhonghua 11 (*Orazy sativa* L.ssp japonica; *See,* Zhong-Hai Ren et al., 2005, A rice quantitative trait locus for salt tolerance encodes a sodium transporter. Nature Genetics 37, 1141 - 1146; the public can obtain the material from Institute of Botany, the Chinese Academy of Sciences) and reverse transcribed to obtain cDNA.

PCR amplification of the obtained cDNA with primer pair 1, and the resultant fragment 1 has a size of about 200bp; Sequencing results confirmed the nucleotide sequence of fragment 1 was the sequence as shown in SEQ ID NO.1; The PCR reaction mixture comprises 2pmol of each primer of primer pair 1, 10μl of PCR Mix (Genestar A112-01), 2μl of cDNA, adding double-distilled water to 20μl. The PCR protocol was: Denaturation at 94°C for 3 minutes, followed by 30 cycles of denaturation at 94°C for 30 seconds, anneal at 55°C for 30 seconds, and extension at 72°C for 30 seconds, and final extension at 72 °C for 10 minutes.

The obtained fragment 1 was mixed with equal molar of pDONR221 vectors(Invitrogen 12535-037) and incubated at 25°C for 1 hour, i.e. BP reaction (Invitrogen 11789-020) was carried out to generate an entry vector. The entry vector was transformed into *Escherichia coli* DH5α by heat shock. The transformed cells were spread on a LB medium plate plus 50mg/L of kanamycin for overnight to obtain transformants (simple principle: original plasimid pDONR221 comprises a lethal gene ccd B, so transformants can't survive; only when ccd B gene is replaced by exogenous fragment, bacterial colonies can survive).

| | | | |
|---|---|---|---|
| BP reaction system: | Fragment 1/ Fragment 2 | 100ng/μl | 1μl |
| | pDONR/Zeo vector | 100ng/μl | 1μl |
| | BP Clonase II Enzyme mix | | 2μl |
| | TE Buffer (PH=8.0) | | 6μl |

BP reaction procedure: incubation at 25 °C for 1 hour, then 1μl of protease K was added and mixed, followed by incubation at 37°C for 10 minutes.

Colony PCR of the transformants was carried out using Primer pair 1. Single colonies which could obtain PCR product of 200bp were preserved as positive clone.

Plasmid was extracted from positive clone and then sequenced. Plasmids extracted from the confirmed positive clone represents the vector obtained by inserting the sequence as shown in SEQ ID NO.1 into pDONR221 vector and was named pDONR/osc8-1.

2. Obtaining Gateway terminal vector pH7GWIWGII-osc8-1 (i.e. RNA interference vector of OsOSC8).

The pDONR/osc8-1 vectors obtained by the above mentioned step 1 were mixed with equal molar ofpH7GWIWG2 (II) vectors (Damme et al., Somatic Cytokinesis and Pollen Maturation in Arabidopsis Depend on TPLATE, Which Has Domains Similar to Coat Proteins. plant cell, 2006, 18: 3502-3518. The public can obtain the material from Institute of Botany, the Chinese Academy of Sciences) and incubated at 25 °C for 1 hour, i.e. LR reaction (invitrigen 11791-020). The mixture was transformed into *Escherichia coli* DH5α by heat shock and the transformed cells were spreaded on a plate plus 100mg/L of spectinomycin. Incubate at 37°C overnight to obtain respective transformants (the principle is the same as BP reaction).

| | | | |
|---|---|---|---|
| LR reaction system: | pH7GWIWG2 (II) | 100ng/μl | 1μl |
| | pDONR/osc8-1 | 100ng/μl | 1μl |
| | LR Clonase Reaction Buffer | | 4μl |
| | TE Buffer (PH=8.0) | | 10μl |
| | LR Clonase enzyme mix | | 4μl |
| | | | 20μl |

LR reaction procedure: incubation at 25°C for 1 hour, then 2μl of protease K was added and mixed followed by incubation at 37°C for 10 minutes.

Plasmid was extracted from single colonies of transformants and then sequenced. Plasmid in confirmed single clone of transformants was named pH7GWIWGII-osc8-1 and contained the sequence as shown in SEQ ID NO.1 which was inserted into the pH7GWIWG2 (II) vector in forward direction and reverse directions. It was an RNA interference vector.

II Obtaining fertility-lowered plants by using RNA interference vector of OsOSC8

1. Obtaining recombinant *Agrobacterium tumefaciens*

The RNA interference vector pH7GWIWGII-osc8-1 obtained by the above step I was introduced into *Agrobacterium tumefaciens* strain EHA105 (K L Piers et al., 1996, Agrobacterium tumefaciens-mediated transformation of yeast. PNAS February 20, vol. 93 no. 4 1613-1618; the public can obtain the material from Institute of Botany, the Chinese Academy of Sciences) by means of electroporation under a voltage of 1800V to obtain transformants.

The resultant mixture was cultured at 28°C for two days and then single clone were picked up to perform PCR amplification (using primer pair 1). Colonies with the expected fragments of 200bp are positive clone named EHA105/pH7GWIWGII-osc8-1 and preserved in 15% of glycerol at -80°C.

2. Obtaining RNA interference transgenic plants

1) Cultivation of rice calli

a) Sterilized $ddH_2O$, 70% ethanol, 1% corrosive sublimate and sterilized 100ml-triangular flasks were placed in hood and then shut the hood with glass window. Ultraviolet radiation was performed after 30 minutes.
b) Seeds of rice Zhonghua 11 (hereinafter refers to wild-type rice) were put into a sterilized triangular flask and rinsed by sterilized $ddH_2O$ for 3 times to remove floating seeds and impurities on the surface of the seeds.
c) Following discard of $ddH_2O$, the seeds were sterilized by 70% ethanol for 8 minutes. The triangular flask was shaked from time to time to facilitate a complete sterilization.
d) Following discard of 70% ethanol, the seeds were sterilized by 1% corrosive sublimate for 8 minutes. Do not use too much corrosive sublimate, only submerging the surface of the seeds.
e) Following discard of 1% corrosive sublimate, the seeds were rinsed by sterilized $ddH_2O$ for 4 times and then added appropriate amount of sterilized $ddH_2O$ (liquid level is 1 cm higher than the surface of the seeds) followed by sealing with a sealing film and immersion for 12 hours.
f) Mature embryos of rice seeds were cut off under sterile conditions and inoculate in induction medium NB2 and cultured in dark at 25 °C for 3-4 weeks.
g) Calli growing from mature embryos were cut off and transferred to subculture medium NB1 and cultured in dark at 25 °C for 2 weeks.
h) Calli in good condition were cut into pieces of callus with a size of a mung bean and transferred to NB1 medium and cultured in dark at 25 °C for 4 days.

2) Preparation and transformation of *Agrobacterium tumefaciens*

a) EHA105/pH7GWIWGII-osc8-1 was inoculated in YEB+RIF+SPE liquid medium (i.e. YEB medium containing 25mg/L of rifampicin and 100mg/L of spectinomycin) in a proportion of 1:100 and cultured at 28°C, 230rpm for 23 hours.
b) The cultured bacterial suspension was then inoculated in YEB+RIF+SPE liquid medium in a proportion of 1:50 and cultured at 28°C, 230rpm till reach an OD600 of 0.5. The bacterial suspension was collected into a 50ml-sterilized centrifuge tube and precipitated by centrifugation at 4000 g for 5 minutes. The supernatant was discarded.
c) Collected bacteria were resuspended with 50ml AAM-AS medium in a sterilized 100ml-triangular flask and shaked in a shaker for 45 minutes to make the bacteria uniformly dispersed in medium.
d) Calli obtained by the above 1) were precultured for 4 days and then immersed in resuspended bacterial suspension for 5-10 minutes. The triangular flask was shaked occasionally.
e) Discard the bacterial suspension. Calli were placed on sterile filter papers and transferred to co-culture medium NB2C (a layer of sterile filter paper with appropriate size was placed on the surface of the medium in advance) after bacterial suspension was absorbed by filter papers. Calli were cultured in dark at 25 °C for 4 days to obtain calli which were co-cultured for 4 days.

3) Selection and regeneration of positive calli

a) Calli which were co-cultured for 4 days in the above 2) were transferred to NB1 medium containing hygromycin (20mg/L) and Timentin (225mg/L) and cultured in dark at 25 °C for 2 weeks to select positive transformants.
b) Calli infected with bacteria in the first selection were discarded and the rest were transferred to NB1 medium containing hygromycin (20mg/L) and Timentin (180mg/L) and cultured in dark at 25 °C for 2 weeks to perform the second selection.
c) Calli infected with bacteria in the second selection were discarded and the rest were transferred to NB1 medium containing hygromycin (50mg/L) and Timentin (180mg/L) and cultured in dark at 25 °C for 2 weeks to perform the third selection.

d) When calli grew to 0.5-0.8cm, check them under fluorescence microscope. Select calli with green fluorescence. The selected calli were transferred to regeneration medium DR1 and cultured in dark for 1 week followed by culture in light (at 23 °C under a 12/12 h (day/night) photoperiod with light supplied at an intensity of 5000lux) for 1 week.

e) Regenerated seedlings or calli were transferred to regeneration medium DR2 all together and cultured in light (the same as above) for 2 weeks.

f) Regenerated seedlings were transferred to grass carbon soil when seedlings grew to about 8cm and cultivated at 28 °C in glass green house. 30 RNA interference transgenic rice plants of T0 generation were obtained.

pDONR 221vectors and pH7GWIWG2 (II) vectors were mixed for LR reaction using the same method to obtain RNA interference blank vector. Then RNA interference blank vectors were transformed into wild-type rice by *Agrobacterium tumefaciens* to obtain blank vector transgenic rice RNAi-CK-3.

Part of the above used medium are as follows in tables 1-4:

Table 1 Formula of medium

| Components | NB1 (1L) | NB2 (1L) | NB2C (1L) |
|---|---|---|---|
| N6 major element (10×) | 100ml | 100ml | 100ml |
| Fe salt (100×) | 10 ml | 10 ml | 10 ml |
| inositol (100×) | 10 ml | 10 ml | 10 ml |
| B5 organics (100×) | 10 ml | 10 ml | 10 ml |
| B5 microelement (1000×) | 1 ml | 1 ml | 1 ml |
| 2, 4-D (1mg/ml) | 0.5 | 2ml | 2ml |
| L-Glutamine | 0.5g | 0.5g | 0.5g |
| L-Proline | 0.5g | 0.5g | 0.5g |
| CH( acid hydrolysis of casein) | 0.3g | 0.3g | 0.3g |
| Sucrose | 30g | 30g | 30g |
| Glucose | | | 10g |
| AS (50mg/ml) | | | 2ml |
| Adjust pH value to | 5.8 | 5.8 | 5.2 |

Table 2 Formula of AAM-AS medium

| AAM-AS major element（10×）1L | AAM-AS amino acid（100×）100ml | AAM-AS vitamin（1000×）100ml |
|---|---|---|
| $CaCl_2.2H_2O$ 1.5g | Glutamine 8.76g | Glycine 0.75g |
| $KH_2PO_4$ 1.2g | Aspartic acid 2.66g | Thiamine hydrochloride 0.01g |
| $MgSO_4.7H_2O$ 2.5g | Arginine 1.74g | Pyridoxine hydrochloride 0.05g |
| KCl 29.5g | (dissolve separately and then mix) | nicotinic acid 0.05 g |

| Components | DR1（1L） | DR2（1L） | AAM-AS（1L） |
|---|---|---|---|
| MS major element（10×） | 100ml | 100ml | AAM-AS major element（10×）100ml |
| Fe salt（100×） | 10 ml | 10 ml | AAM-AS amio acid（100×） 10ml |
| inositol（100×） | 10 ml | 10 ml | inositol（100×） 10ml |
| MS organics（100×） | 10 ml | 10 ml | AAM-AS vitamin（1000×） 1ml |
| MS microelement（1000×） | 1ml | 1ml | B5 microelement（1000×）1ml |
| 6-BA（1mg/ml） | 1ml | 1ml | CH( acid hydrolysis of casein ) 0.5g |
| KT（1mg/ml） | 500μl | 500μl | Sucrose 30g |
| NAA（1mg/ml） | 250μl | 500μl | 2，4-D（1mg/ml） 500μl |
| ZT（0.2mg/ml） | 1ml | 1ml | AS（50mg/ml） 1ml |
| CH( acid hydrolysis of casein ) | 0.3 | 0.3 | |
| Sucrose | 30 | 30 | |
| Sorbitol | 30 | 30 | |
| Adjust pH value to | 5.8 | 5.8 | 5.2 |

Table 3 Formula of major element

| major element (10×) 1L | | |
|---|---|---|
| Components | N6 major element | MS major element |
| $CaCl_2.2H_2O$ | 1.66g | 4.4g |
| $NH_4NO_3$ | | 16.5g |
| $KNO_3$ | 23.8g | 19g |
| $(NH_4)_2SO_4$ | 4.63g | |
| $KH_2PO_4$ | 4g | 1.7g |
| $MgSO_4.7H_2O$ | 1.85g | 3.7g |

Table 4 Formula of microelement

| microelement (1000×) 1L | | |
|---|---|---|
| Components | B5 microelement | MS microelement |
| $MnSO_4.4H_2O$ | 10 g | 22.3g |
| $ZnSO_4.7H_2O$ | 2g | 8.6g |
| $H_3BO_3$ | 3g | 6.2g |

(continued)

| microelement (1000×) 1L | | |
|---|---|---|
| Components | B5 microelement | MS microelement |
| KI | 0.75g | 0.83g |
| $Na_2MoO4.2H_2O$ | 0.25g | 0.25g |
| $CuSO_4.5H_2O$ | 0.025g | 0.025g |
| $CoCl_2.6H_2O$ | 0.025g | 0.025g |

Table 5 Formula of organics

| Organics (100×) 200ml | | |
|---|---|---|
| Components | B5 organics | MS organics |
| Glycine | | 0.04g |
| Thiamine hydrochloride | 0.2 g | 0.008g |
| Pyridoxine hydrochloride | 0.02 g | 0.01g |
| nicotinic acid | 0.02 g | 0.01 g |

Table 6 Other formulae

| Fe salt (100×) 200ml | | inositol (100×) 200 ml |
|---|---|---|
| Liquid A | $FeSO_4.7H_2O$ 100ml (27.8 mg/ml) | inositol 2g $ddH_2O$ 200ml |
| Liquid B | Na-EDTA 100ml (37.3 mg/ml) | |
| Mix A and B | | |

3. Western blot analysis of RNA interference transgenic rice

When RNA interference transgenic rice of the T0 generation obtained by the above 2 was at the booting stage, protein was extracted from young panicles at the vacuolated microspore stage and analyzed by western blot (antibody was polyclonal antibody of OsOSC8 protein which was isolated from serum of rabbits immunized with OsOSC8 protein. The antibody can be monoclonal antibody of OsOSC8 protein made by Shanghai Abmart Company). Measure the content of OsOSC8 protein.

The result is shown in Figure 1, wherein Zh11 represents wild-type rice, RNAi-9, RNAi-12, RNAi-20 and RNAi-21 represent RNA interference transgenic rice of the T0 generation and RNAi-CK-3 represents blank vector transgenic rice. It can be seen that expression of OsOSC8 in transgenic lines RNAi-9, RNAi-12 and RNAi-20 was reduced obviously, whereas expression of OsOSC8 in the control line RNAi-CK-3 is equal to wild-type rice.

The results show that RNAi-9, RNAi-12, RNAi-20 and RNAi-21 are positive RNA interference transgenic rice of the T0 generation which are mutants obtained by silencing of the OsOSC8 gene in rice by means of RNA interference.

4. Statistics of setting percentage of RNA interference transgenic rice

Setting in natural condition: RNA interference transgenic rice of the the T0 generation which were named as RNAi-9, RNAi-12, RNAi-20 and RNAi-21 and identified by the above 3 were cultivated in glass green house with natural lighting and maintained at 18°C/25°C (night/day). Growth conditions: temperature 18°C/30°C (night/day), humidity 30%-50%, natural lighting. Setting percentage of each panicle was analyzed statistically 4 weeks after flowering of RNA interference transgenic rice of the T0 generation and the results were compared to blank vector transgenic rice RNAi-CK-3 and wild-type rice ZH11. 5 panicles of each transgenic plant were analyzed and the results were mean value±standard deviation.

Setting percentage= the percentage of plump seeds in total seeds (plump seeds+ empty seeds);

The results are shown in Figure 2.

Setting in natural condition, the setting percentages of wild-type rice ZH11, RNA interference transgenic rice of the T0 generation RNAi-9, RNAi-12, RNAi-20 and RNAi-21 are 93.5±5.3%, 42.1±15.3%, 37.9±3.9%, 18.6±16.1% and 81.1±6.1% respectively.

There is no significant difference in setting percentage between blank vector transgenic rice RNAi-CK-3 and wild-type rice.

Thus the natural setting percentages of RNA interference transgenic rice of the T0 generation fall below 40%, wherein that of RNAi-20 fall below 20% and the natural setting percentage of wild-type rice is above 80%.

It indicates that fertility of RNA interference transgenic rice of the T0 generation obtained by silencing of the OsOSC8gene in rice by means of RNA interference is lowered compared to wild-type rice. Sterile transgenic rice can be obtained by selecting more transgenic plants.

Example 2 Obtaining fertility-lowered mutant by utilizing TILLING technology

I Selection of fertility-lowered mutant by utilizing TILLING technology

1. Mutating seeds of a target plant; Designing primer pairs for specifically amplifying genes encoding triterpene synthase in the target plant.

1) Mutating seeds of a target plant

20,000 grains of seeds of Zhonghua 11 rice were immersed in 2mM aqueous solution of sodium azide at 25 °C for 6 hours to obtain mutated seeds.

2) Primer design

Primers were designed according to OsOSC8 gene sequence encoding triterpene synthase in rice. The primer can specifically amplify said gene. The sequences were:

Primer pair 2:

Sense primer OsOSC8T1F: GAGGTCAAGTCGTCTTCTGCAATTA (SEQ ID NO. 6);
Antisense primer OsOSC8T1R: ATTTGTCTGCGCTCTGCACATG (SEQ ID NO. 7);

Primer pair 3:

Sense primer OsOSC8T13F: GCTTAAAGGTAAATTTCAGGCTTCC (SEQ ID NO. 8);
Antisense primer OsOSC8T13R: CGATCAGAATCAATTAAACCCAGAC (SEQ ID NO. 9);

Primer pair 4:

Sense primer OsOSC8T17F: TCATCCTTAGATTAATTAGCCGACA (SEQ ID NO. 10);
Antisense primer OsOSC8T17R: CATAAGGATCTCATAAAATCGACCA (SEQ ID NO. 11);

Each of the above primer pairs were labeled with fluorescently labeled probes of different wavelengths. Fluorescently labeled probes having different wavelengths are fluorescent dye DY-682 with a wavelength of 682nm (Eurofins DNA Campus Ebersberg, Germany) and fluorescent dye DY-782 with a wavelength of 782nm (Eurofins DNA Campus Ebersberg, Germany).

The 5' end of all sense primers were labeled by DY-682 fluorescence (DY-682), while the 5' end of all antisense primers were labeled by DY-782 fluorescence (DY-782).

2. Cultivation

The above obtained mutated seeds were rinsed with water and cultivated in field to obtain the first generation of mutation M1. Self-cross of the first generation of mutation M1 to obtain the second generation of mutation M2. Then self-cross of the second generation of mutation M2. Harvest and preserve seeds of the third generation of mutation M3.

3. Extraction of DNA and construction of gene pool

Seeds of the second generation of mutation M2 were harvested. Random 12 plants of each line of M2 were planted and genomic DNA of individual plant of the second generation of mutation M2 was extracted and preserved at -20°C for subsequent use. Genomic DNAs from 4 individual plants of M2 were mixed (mixed with equal quantity) to obtain a DNA pool. Detect the quality and measure the concentration of DNA and then the DNA is uniformally mixed.

4. PCR amplification

PCR amplification of the above DNA pools with primer respective pair 2, 3, and 4 to obtain 3 types of PCR amplified products. Procedure and system of PCR amplification were as follows:

Table 7 PCR system

| Master mix | 10ul for each reaction | × 104 |
|---|---|---|
| dNTP (2.5mM) | 0.8μl | 83.2μl |
| 10×Buffer | 1μl | 104μl |
| Sense primer with fluorescence label (10μM) | 0.096μl | 9.984μl |
| Sense primer without fluorescence label (10μM) | 0.064μl | 6.656μl |
| Antisense primer with fluorescence label (10μM) | 0.128μl | 13.312μl |
| Antisense primer without fluorescence label (10μM) | 0.032μl | 3.328μl |
| EX Taq | 0.1μl | 10.4μl |
| ddH$_2$O | 5.78μl | 601.12μl |
| DNA | 2μl | |

Procedure: 95°C 2min
94°C 20s
65°C 30s
72°C 1.5min(varies according to different fragments) } 35 cycles
72°C 5min
95°C 10min

70°C 20s (-0.3°C/cycle) 70cycles
15°C 5min

PCR product was placed in dark and on ice after PCR reaction.

5. Enzyme digestion

PCR product of each DNA pool obtained by the above 4 was digested with CEL I enzyme to obtain products of enzyme digestion. The system of enzyme digestion was shown in Table 8:

Table 8 System of enzyme digestion of CEL I

| Master mix | 15ul for each reaction | × 104 |
|---|---|---|
| PCR products | 9ul | |
| CEL I | 0.1μl | 10.4ul |

(continued)

| Master mix | 15ul for each reaction | × 104 |
|---|---|---|
| 10×CEL I Buffer | 1.5μl | 156μl |
| $ddH_2O$ | 4.45ul | 462.8ul |
| Procedure: Enzyme digestion at 45 °C for 15 minutes. | | |

6. Electrophoresis analysis

1) Electrophoresis

Enzyme-digested products of each PCR product corresponding to each DNA pool obtained by the above 5 were purified and electrophoresed, wherein a sample in each lane was an enzyme-digested product of each PCR product of a DNA pool.

Purification: 15ul enzyme digested product, 20ul ddH2O, 5ul 0.225M EDTA and 60ul isopropanol were added into a 96 wells plate. The plate was covered by a silica gel cover and the mixture was turned upside down for 30 times and then incubated at room temperature for 15 minutes followed by centrifugation at 4°C, 3000rpm for 30 minutes. Supernatant was discarded after centrifugation and the 96 wells plate was inverted on a paper towel followed by brief centrifugation at 4°C, 3000rpm for 10 seconds. 100ul 75% ethanol was added to the precipitate in the 96 wells plate. The plate was covered by a silica gel cover and the mixture was turned upside down for 30 times followed by centrifugation at 4°C, 3000rpm for 20 minutes. Repeat the steps twice. The sample plate was dried in a ventilation system for 2min. The precipitate was dissolved in 5ul loading buffer after it was free of ethanol and shaked for 5 seconds on a votex followed by brief centrifugation for 10 seconds. The DNA was denaturated at 85 °C for 10 minutes.

Electrophoresis:

| | |
|---|---|
| 20ml | 6% gel (commercial available gel) |
| 100ul | AP (-20°C) |
| 25ul | TEMED (4°C) mix immediately |

The sample was denatured at 85 °C for 10 minutes and then placed on ice for 10 minutes.

Table 9 Electrophoresis condition

| | Pre-run | Run |
|---|---|---|
| Volts(v) | 1500 | 1500 |
| Current(mA) | 40 | 40 |
| Power(w) | 40 | 40 |
| Time | 10min | 3hr |
| Temperature (°C) | 45 | 45 |

0.45ul of each sample was added to a 100 wells paper comb rapidly within 10 minutes of prerunning. 1ml 1% Ficoll was added into each comb well without TBE and the comb with samples was inserted into the wells rapidly. Li-COR 4300 was turned on and ran for 3 hours. The runtime varies according to the size of amplified fragments.

2) Data analysis

Photos of the above electrophoresis results were processed by Adobe Photoshop 8.0. Mode was changed from 16 channels to 8 channels. The photos were rotated and set as pictures having a width of 20cm and a length of 27cm. Defined ratio was cancled and then brightness and contrast were adjusted. The photos were finally saved in JPEG format and analyzed using Gelbuddy. The photos were observed under 682nm and 782nm.

Enzyme-digested products of each DNA pool were analyzed by electrophoresis to identify fertility-lowered mutants or sterile mutants. If enzyme-digested products of said DNA pool generated bright dots under all wavelengths of different fluorescently labeled probes, individual plants of M2 (with numer n) of said DNA pool contained or might contain fertility-lowered mutants or sterile mutants; If enzyme-digested product of said DNA pool did not generate bright dots under all wavelengths of different fluorescently labeled probes, individual plants of M2(with numer n) of said DNA pool did not contain or might not contain fertility-lowered mutants or sterile mutants. Said fertility-lowered mutants were plants whose fertility were lowered than said target plant.

The aforementioned method can be used directly to identify a point mutation in a triterpene synthase encoding gene. If enzyme-digested product of said DNA pool generated bright dots under all wavelengths of different fluorescently labeled probes, the triterpene synthase encoding gene in said DNA pool had or might have a point mutation. If enzyme-digested product of said DNA pool did not generate bright dots under all wavelengths of different fluorescently labeled probes, the triterpene synthase encoding gene in said DNA pool did not have or might not have a point mutation.

Partial results are shown in Figure 3, wherein the left picture represents result of DY-682 and the right picture represents result of DY-782. The arrows point to mutants. Arrow 1 points to one mutant, while arrow 2 points to another mutant. It can be seen that both enzyme-digested products of two DNA pools in two lanes generate bright dots under DY682 and DY782 which indicates that 4 individual plants of M2 of these two DNA pools have sterile mutants or fertility-lowered mutants.

In order to further identify which individual plant was a mutant, 4 individual plants of anyone of the above DNA pools which were identified as sterile mutants or fertility-lowered mutants were used as templates with genomic DNA (mixed with equal quantity) of wild-type rice respectively. Steps 4-5 were repeated to obtain enzyme-digested products of M2 individual plant. Enzyme-digested products of M2 individual plant were purified and electrophoresed, wherein a sample in each lane was an enzyme-digested product of each PCR products of genomic DNA of each M2 individual plant; each lane corresponded to genomic DNA of a M2 individual plant.

If enzyme-digested products of said M2 individual plant generated bright dots under all wavelengths of different fluorescently labeled probes, said M2 individual plant was or might be a sterile mutant or fertility-lowered mutant. If enzyme-digested products of said M2 individual plant did not generate bright dots under all wavelengths of different fluorescently labeled probes, said M2 individual plant was not or might not be a sterile mutant or fertility-lowered mutant.

The specific primer pair corresponding to the enzyme-digested products of PCR product of said lane was identified simultaneously (according to the specific primer pair corresponding to the enzyme-digested product of PCR product of the lane in which bright dots generated, the corresponding specifically amplified product was identified) and used as verification primers subsequently.

Total 3 fertility-lowered mutants of M2 individual plants named P34E8, 4928 and 1708 were selected. Said mutants were also mutants of triterpene synthase encoding gene *OsOSC8*. The corresponding gene-specific primer pairs of the mutants were as follows: primer pair 2 for selection of P34E8 and 4928 and primer pair 3 for selection of 1708.

II Verification of fertility-lowered lines selected by TILLING technology

1. Molecular verification of point mutation of triterpene synthase encoding gene

RNA was extracted from the above selected 3 fertility-lowered mutants of M2 individual plants P34E8, 4928 and 1708 and reverse-transcribed into cDNA. PCR amplify the cDNA with primer pair 1. The PCR products were sequenced and the mutated sites of the 3 mutants P34E8, 4928 and 1708 were identified as shown in the following table 10:

Table 10 Mutated sites of each mutant and change of amino acid

| Mutant No. | Change of Nucleotide | Change of Amino Acid |
|---|---|---|
| P34E8 | G764A | W255- |
| 4928 | G809A | G270E |
| 1708 | G1431A | R477K |

Sites of amino acid and nucleotide in the above table correspond to the sites of the sequences of protein (amino acid sequence as shown in SEQ ID NO.2) and gene (nucleotide sequence as shown in SEQ ID NO.5) of OsOSC8.

Amino acid sequence of P34E8 was obtained by mutating the amino acid residue at the 255 position of N' end of the sequence as shown in SEQ ID NO.2 from Trp to termination codon. Nucleotide sequence of P34E8 was obtained by mutating the nucleotide residue at the 764 position of 5' end of the sequenceas shown in SEQ ID NO.5 from G to A.

Amino acid sequence of 4928 was obtained by mutating the amino acid residue at the 270 position of N' end of the sequence as shown in SEQ ID NO.2 from Gly to Glu. Nucleotide sequence of 4928 was obtained by mutating the nucleotide residue at the 809 position of 5' end of the sequence as swhown in SEQ ID NO.5 from G to A.

Amino acid sequence of 1708 was obtained by mutating the amino acid residue at the 477 position of N' end of the sequence as shown in SEQ ID NO.2 from Gly to Lys. Nucleotide sequence of 1708 was obtained by mutating the nucleotide residue at the 1431 position of 5' end of the sequence as shown in SEQ ID NO.5 from G to A.

The mutant P34E8 selected above was a mutant strain of OsOSC8 which was deposited in China General Microbiological Culture Collection Center (CGMCC for short, address: No.3, Courtyard No. 1, West Road Beichen, Chaoyang District, Beijing) on 28 May 2012 with a deposit Number of CGMCC No. 6150. The classification and nomenclature is rice *Oryza sativa.*

2. Phenotype identification of sterile lines selected by TILLING

Seeds of M3 of the 3 fertility-lowered mutants P34E8, 4928 and 1708 were used in the following experiments.

1) Statistics of setting percentage of the mutants

(1)Seeds of the 3 fertility-lowered mutants P34E8, 4928 and 1708 obtained in the above I were planted at 18°C/25°C (night/day) in glass green house with natural lighting. Growth conditions: temperature 18°C/30°C (night/day), humidity 30%-50%, natural lighting.

(2)Setting percentage of florets can be observed 2 weeks after rice flowering; 5 panicles were analyzed and experiments were repeated 3 times. The results were mean value±standard deviation. The control was wild-type rice.

As a result, setting percentage of wild-type rice is 94.81%±1.34%; setting percentage of mutant P34E8 is only 1.85%±0.49%; setting percentages of mutant 4928 and mutant 1708 are 4.38%±0.24% and 3.87%±0.36% respectively. Thte results indicate that the fertility of selected mutants P34E8, 4928 and 1708 is lower than the wild-type rice.

2) Fertility phenotype identification of mutants

Seeds of the fertility-lowered mutant P34E8 (S6) obtained in the above I was planted at 18°C/25°C (night/day) in glass green house with natural lighting. Growth conditions: temperature 18°C/30°C (night/day), humidity 30%-50%, natural lighting.

(1) Tillering

Mutant P34E8 and wild-type rice were observed during vegetative growth stage (50-70 days after seeding). Both of them grew normally and had 3-5 tillers, wherein mutant P34E8 did not show abnormal characters.

(2) Floral organ

14 weeks after seeding, mutant P34E8 (S6) and wild-type rice began heading and flowering. Inflorescence, shape of florets and number and size of floral organs of mutant P34E8 and wild-type rice were observed.

As a result, mutant P34E8 (S6) had normal panicles and oblong florets with a complete set of floral organs which contained one lemma, one glumelle, six stamens, one pistil (with a two-split feathery stigma) and two lodicules. The size of floral organs of mutant P34E8 (S6) developed normally (Figure 4A, B, C, D) and didn't have changes compared to wild-type rice.

I2-KI staining (KI 3g, $I_2$ 1g, diluted to 300ml) of pollens of mutant P34E8 (S6) and wild-type rice were carried out and observed under a microscope (microscope model: OLYMPUS BX51) after 5 minutes. The result of staining showed that pollens of the mutant and wild-type rice were blue black in $I_2$ which indicated that accumulation of starch thereof was normal (Figure 4E, F).

Alexander staining (refer to Alexander MP., 1969, Stain Technol, 44:117-122) of pollens of mutant P34E8 (S6) and wild-type rice were carried out. The staining solution was prepared as 50x Master solution. 10ml absolute ethanol, 1ml 1% malachite green (prepared with 95% ethanol), 5g phenol, 5g chloral hydrate, 5ml 1% acid fuchsin solution, 0.5ml 1% orange G aqueous solution, 2ml glacial acetic acid and 25ml glycerol were mixed and adjusted to 100ml with distilled water and stored in a brown bottle. The Master solution was diluted before use according to a ratio of Master solution: distilled water = 3:47 (v: v). Staining method was the same to I2-KI staining. The result of staining showed that pollens of the mutant and wild-type rice were dyed purple red which indicated that pollens were viable (Figure 4 G, H).

*In vitro* germination of pollens of mutant P34E8 (S6) and wild-type rice were carried out to detect the vitality thereof. In particular, pollens were cultivated in a culture medium containing 20% sucrose, 10% PEG4000, 40mg/L boric acid, 3mmol/L calcium nitrate, 3mg/L vitamin B1. Detection Method: 2-3 drops of culture medium were droped on a glass slide, and then anthers of a floret which just opened and was about to loose powder were placed in the culture medium and broken by a pointed tweezer. Massive anther walls were removed and the sample was covered with a coverslip. Samples were placed in a big culture dish covered with wet gauze (moisturizing) and incubated at 30°C in an incubator and observed after 30 minutes.

The result of observation indicated that the highest pollen germination rate of wild-type rice was 82.8% and the highest pollen germination rate of mutant P34E8 (S6) was 80%. Therefore, vitality of pollens of mutant was good in *in vitro* germination experiment (Figure 5).

(3) Ability of pollens to adhere to stigmas

*In vivo* germination of pollens of mutant P34E8 (S6) and wild-type rice were carried out to detect the vitality on stigmas thereof. Callose is β-1, 3 - glucan which is usually distributed in sieve tube, newly formed cell walls, pollens and pollen tubes of higher plants. It can give out yellow to yellow-green fluorescence under UV excitation after dyed with water-soluble aniline blue. Therefore, germination of pollens on stigmas, status of pollen tube development, as well as state of deposition of callose on the surface of stigmas, etc. can be observed by placing pollinated ovaries after dyed with aniline blue under fluorescence microscope and then to determine whether the pollens were compatible with the stigmas. Method (Refer to Endo M et al., 2009, Plant Cell Physiol, 50:1911-1922): Rice florets at different time after pollination were fixed in Kano's stationary liquid (absolute ethanol: glacial acetic acid = 3: 1 ( v: v)). The volume of the fixation solution is at least 10 times of the materials. Fix for 30 miniutes to 2 hours (should not over 24 hours, or the tissue will become fragile). Then rinse the materials successively by 95% ethanol for 5 minutes, 70% ethanol for 5 minutes and distilled water for 5 minutes to remove glacial acetic acid. Then florets were treated with 1N NaOH at 60°C for 30 minutes and the softened materials were rinsed by distilled water 3 times (to remove most sodium hydroxide. As the materials were brittle now, carefully operate), each time for 5 minutes. 0.1% water-soluble aniline blue dye solution (Aniline blue, Sinopharm Chemical Reagent Co., Ltd., prepared with 0.1M potassium phosphate aqueous solution) was dropped after rinse. Materials were just immerged in dye solution and dyed in dark for 1 hour or so. A drop of 50% glycerol was dropped on a slide. Florets after dyeing were taken out by a tweezer. Stigmas were isolated. Two-split feathery stigmas were placed roughly straight and covered by a coverslip. Styluses were spreaded by gentle pressure (not too hard) and then observed under UV Fluorescence microscope (Microscope model: OLYMPUS BX51).

The result of observation was shown in Figure 6. It indicated that 5 minutes after pollination patial pollens of wild-type rice adhered to stigmas and more and more pollens adhered to stigmas with time extending. 20 minutes after pollination pollen tubes have began to extend and 60 minutes after pollination some pollen tubes have entered the ovule. However, in the corresponding time, little or no pollens of mutants adhered to stigmas, which further proved that reduction of fertility of mutant P34E8 (S6) was caused by reduction of ability of pollens to adhere to stigmas or the fact that pollens did not adhere to stigmas.

In order to prove that the condition that pollens of mutant P34E8 (S6) did not adhere to stigmas was caused by pollen grain itself or by changes of stigmas, mutant P34E8 (S6) was hybridized with wild-type. Uncracked pollens of wild-type were pollinated on stigmas of mutant and uncracked pollens of mutant were pollinated on stigmas of wild-type. 20 minutes and 60minutes after pollination stigmas were fixed in Kano's fixation solution and stained with aniline blue. Adhesion and germination of pollens on stigmas were detected.

The result of detection was shown in Figure 7. It indicated that pollens of wild-type could adhere to stigmas of homozygous mutant, germinate and enter the ovule, while pollens of mutant could not adhere to stigmas of wild-type. This indicated that the pollens of mutant failing to adhere to stigmas was caused by pollen itself rather than stigmas.

The above experiments further proved that fertility of mutant P34E8 (S6) was lowered compared to wild-type rice and it was caused by reduction of ability of pollens to adhere to stigmas or the fact that pollens did not adhere to stigmas.

Mutants 4928 and 1708 were identified using the same method and the result had no significant difference with that of mutant P34E8 (S6). Fertility of both mutants was lowered than wild-type rice, and reduction of fertility was caused by the fact that pollens did not adhere to stigmas.

The above results indicate that mutation of triterpene synthase encoding genes can result in reduction of rice fertility. Thus, TILLING selection method can be used to obtain fertility-lowered rice, even sterile rice.

Example 3 Restoring fertility or improving fertility

Seeds of M3 generation of fertility-lowered mutant P34E8 were used in the following experiments.

Moisturizing treatment: T0 generation of RNA interference transgenic rice of RNAi-9, RNAi-12, RNAi-20 and RNAi-21 obtained in the above example 1 and mutant P34E8 (S6) obtained in example 2 were seeded. During rice anthesis (anthesis is from the $80^{th}$ day to the 100th day after seeding), humidity for growth of rice inflorescence was maintained at 80-100% and returned to natural humidity (natural humidity was 40-60%) after one week. Specifically, the method of maintaining humidity for growth of rice inflorescence was as follows: Wrapping the whole rice inflorescence with a plastic bag (standard: length×-width=27cm×15cm) and clipping the plastic bag at the opening with a paper clip; or using preservative film to cover the whole inflorescence. There was no need to use a paper clip to clip preservative film since preservative film sticked together easily.

Rice inflorescence flowered from the top to the bottom and the anthesis of the whole inflorescence was about one week. Wrapping plastic bag and preservative film must be removed in time after flowering of the whole inflorescence because a lot of vapor was gathered during wrapping and too high humidity was not good for the following fructification.

Setting percentages of 5 panicles were analyzed and the experiment was repeated 3 times. The results were mean value±standard deviation. The control was wild-type rice ZH11 (WT).

Setting percentages of wild-type rice ZH11 and T0 generation of RNA interference transgenic rice of RNAi-9, RNAi-12, RNAi-20 and RNAi-21 obtained in example 1 after moisturizing treatment were shown in Figure 2. Setting percentages of wild-type rice ZH11 and T0 generation of RNA interference transgenic rice of RNAi-9, RNAi-12, RNAi-20 and RNAi-21 obtained in example 1 after moisturizing treatment were 89.9±1.8%, 78.9±11.3%, 60.2±3.9%, 85.2±16.18% and 80.3±3.9% respectively; In contrast, the setting percentages of RNAi-9, RNAi-12, RNAi-20 and RNAi-21 without moisturizing treatment were only 42.1±15.3%, 37.9±3.9%, 18.6±16.1 and 81.1±6.1%, respectivley.

Fruiting phenotype of mutant P34E8 (S6) after moisturizing treatment was shown in Figure 8. It can be seen in the figure that setting percentage of P34E8 after moisturizing treatment was greatly increased. Statistical analysis of the setting percentage showed that setting percentage of mutant P34E8 after moisturizing treatment could be 76.25%±3.88%, whereas setting percentage of mutant P34E8 without moisturizing treatment was only 1.85%±0.49%. It indicated that maintaining humidity of rice inflorescence growth could restore fertility or improve fertility.

Example 4 Use of fertility-lowered mutant P34E8 (S6) in breeding

Seeds of M3 of fertility-lowered mutants 4928 and P34E8 (S6) were used in the following experiments.

Preparation of hybrid rice seeds: Grouping for production of hybrid seeds of fertility-lowered mutants 4928 and P34E8 (S6) obtained in example 2 and wild-type rice ZH11 and rice 9311(Jun Yu, Songnian Hu, Jun Wang, Gane Ka-Shu Wong,.... Jian Wang, Lihuang Zhu, Longping Yuan, Huanming Yang. A draft sequence of the rice (Oryza sativa ssp. indica) genome. Science. 296: 79-92, 2002; the public can obtain the material from Institute of Botany, the Chinese Academy of Sciences.) was carried out respectively. Each group contained 30 mutant plants and appropriate amount of wild-type plants were planted at intervals to provide pollens, with 3 replicates.

Meanwhile, fertility-lowered mutants 4928 and P34E8 (S6) were planted respectively in a separate group for selfing. Each group contained 30 plants, with 3 replicates, as control. Artificial supplementary pollination was performed during flowering period (2012.8.20-2012.9.1). The method specifically was: Using an about 2 meters long bamboo pole to pat inflorescence of wild-type rice 5-10 times, to make pollens fly towards inflorescence of mutants, twice a day, at 11:00 and 13:00; the control group was not treated.

After maturation, 30 plants of each hybrid line and selfed line were selected. Setting percentage of one main spike of each plant was analyzed.

The result was shown in Table 11:

Table 11 Setting percentage

| | 9311 | ZH11 | 4928 | P34E8 |
|---|---|---|---|---|
| 4928 | 28.89±3.42% | 31.81±2.79% | 7.45±1.38 | - |
| P34E8 | 22.32±1.71% | 31.34±1.86% | - | 12.84±1.66 |

As seen from the table, selfing setting percentages of mutant 4928 and P34E8 (S6) after in field were 7.45 and 12.84 and hybridization setting percentages were 22.32%-31.81%. Mu yield of production of hybrid seeds was approximately 100-150 kilograms. It indicates that mutants can be used in hybrid rice breeding.

Example 5 Homologous genes of OsOSC8 from other plants and prediction of their functions

I Cloning homologous genes

1)Obtaining of TaOSC1 (from wheat) and HvOSC1 (from barley) Primer design according to EST sequences of hexaploid wheat: 5'primer: 5'-ATGTGGAAGCTCAAGATCGC-3' (SEQ ID NO.12); 3' primer: 5'-TTAGCCAGAGCAAAGTACTAAT -3' (SEQ ID NO.13). Total RNA of flowers of Chinese spring wheat (*Triticum aestivum* L. Jizeng Jia, Zhengbin Zhang, K. Devos,M. D. Gale. Analysis of genetic diversity of 21 chromosomes of Triticum aestivum L. based on RFLP mapping sites. Science in China Series C: Life Sciences. 2001(01); The public can obtain the material from Institute of Botany, the Chinese Academy of Sciences) and barley "Varda" (*Hordeum vulgare* L. Qi X, Niks RE, Stam P, Lindhout P. 1998. Identification of QTLs for partial resistance to leaf rust (Puccinia hordei) in barley. Theor Appl Genet, 96: 1205-1215; The public can obtain the material from Institute of Botany, the Chinese Academy of Sciences.) was used as templates to perform RT-PCR amplification to obtain cDNA. PCR products were detected by 0.8% agarose gel electrophoresis after the reaction finished. The results of detection were shown in Figure 9. The left lane was DNA standard (1kb Ladder), lane Ta was RT-PCR product of wheat, and lanes Hv1 and Hv were RT-PCR products of barley. Bands with a molecular weight of 2-3 kb were obtained and they are consistent with expected size.

RT-PCR products of wheat and barley were sequenced and the results were as follows:

Gene of RT-PCR product of wheat was named *TaOSC1,* having a nucleotide sequence as shown in SEQ ID NO.14 which consists of 2280 bases. The open reading frame (ORF) thereof comprises the 1-2280 bases of 5' end and the protein encoded by the genewas TaOSC1 Amino acid sequence of the protein was shown in SEQ ID NO.15. Homology comparison between TaOSC1 and OsOSC8 showed that similarity of the nucleotide and amino acid sequences were 84.32 % and 85.18% respectively.

Gene of RT-PCR product of barley was named *HvOSC1,* having a nucleotide sequence as shown in SEQ ID NO.16 which consists of 2280 bases. The open reading frame (ORF) thereof comprises the 1-2280 bases of 5' end and the protein encoded by the was HvOSC1. Amino acid sequence of the protein was shown in SEQ ID NO.17. Homology comparison between HvOSC1 and OsOSC8 showed that similarity of the nucleotide and amino acid sequences were 81.58 % and 81.35% respectively.

2) SrOSC1 from sorghum (sorghum bicolor L.) and ZmOSC1 from maize (zea mays L.)

Functions of homologous genes of OsOSC8 in gramineous plants might be very conservative, so putative coding sequences of homologous genes of OsOSC8, namely SrOSC1 and ZmOSC1 were obtained according to the whole genome sequences (http://phyto5.phytozome.net/) of sorghum (sorghum bicolor L.) and maize (zea mays L.).

Amino acid sequence of protein SrOSC1 from sorghum (sorghum bicolor L.) was shown in SEQ ID NO.18 and the gene encoding the protein was shown in SEQ ID NO.19.

Amino acid sequence of protein ZmOSC1 from maize (zea mays L.) was shown in SEQ ID NO.20 and the gene encoding the protein was shown in SEQ ID NO.21.

The above genes can be obtained by artificial synthesis.

II Prediction of functions

TaOSC1 (from wheat), HvOSC1 (from barley), SrOSC1 (from sorghum) and ZmOSC1 (from maize) obtained above were aligned and the result was shown in Figure 10. Red arrowheads show the mutated sites of P34E8, 4928 and 1708. Mutation of these sites in sorghum, maize, wheat and barley may lead to similar restorable sterile phenotype. It can be seen that the method can be used in crossbreeding of sorghum, maize, wheat and barley.

It proved that functions of homologous genes of OsOSC8 in gramineous plants are very conservative.

Thus, according to the studies on OsOSC8 from rice in preceding examples, it can be inferred that silencing the genes of TaOSC1 (from wheat), HvOSC1 (from barley), SrOSC1 protein (from sorghum) and ZmOSC1 protein (from maize) which have high homology with OsOSC8 can also obtain sterile lines.

**INDUSTRIAL APPLICABILITY**

The experiments of the present invention prove that the present invention provides various methods for preparing sterile lines or fertility-lowered lines, including RNA interference or TILLING (Targeting Induced Local Lesions IN Genomes) technology selection. These methods are achieved by silencing expression of gene encoding triterpene synthase. The present invention also provides methods for restoring or improving fertility. Sterile lines prepared by the methods of the present invention establish the basis of rice heterosis and crossbreeding.

## Claims

1. An RNA interference vector obtained by inserting the DNA molecule as shown in SEQ ID NO.1 into a pH7GWIWG2(II) vector.

2. The RNA interference vector according to claim 1, wherein said RNA interference vector is obtained by inserting the DNA molecule as shown in SEQ ID NO.1 into a pH7GWIWG2(II) vector by means of homologous recombination.

3. The RNA interference vector according to claim 1 or 2, wherein said RNA interference vector is prepared according to the following method:

   1) obtaining an intermediate vector by BP reaction between the DNA molecule as shown in SEQ ID NO.1 and a pDONR221 vector;
   2) obtaining an RNA interference vector by LR reaction between said intermediate vector and a pH7GWIWG2(II) vector;

   wherein said DNA molecule as shown in SEQ ID NO.1 is prepared according to the following method: Using rice cDNAs as a template and primer pair A to carry out PCR amplification, the obtained PCR product is the DNA molecule as shown in SEQ ID NO.1;
   said primer pair A consists of the single chain DNA as shown in SEQ ID NO.3 and the single chain DNA as shown in SEQ ID NO.4.

4. A recombinant bacteria or transgenic cell line comprising said RNA interference vector of any one of claims 1 to 3.

5. Use of said RNA interference vector of any one of claims 1 to 3 and said recombinant bacteria or transgenic cell line of claim 4 in cultivating rice sterile lines or reducing rice fertility.

**6.** A method of cultivating a transgenic plant, said method comprising obtaining a transgenic plant by introducing said RNA interference vector of any one of claims 1 to 3 into a target plant; wherein said transgenic plant comprises the following 1) or 2):

1) Sterile transgenic plant; or 2) The fertility of said transgenic plant is lower than that of said target plant;

wherein said target plant is monocot plant; said monocot plant is rice.

**7.** A transgenic plant obtained by the method of claim 6; wherein said transgenic plant is a sterile transgenic plant or fertility-lowered transgenic plant; said plant is monocot plant; said monocot plant is rice.

**8.** A method for cultivating a target plant to a sterile mutant or fertility-lowered mutant, comprising the following steps:

1) Mutating seeds of a target plant; designing primer pair B and fluorescently labeled primer pair B which are used to specifically amplify the gene encoding triterpene synthase from a target plant;
wherein mutated seeds of a target plant are obtained by treating a number of seeds of the target plant with sodium azide;
wherein each primer of said fluorescently labeled primer pair B is labeled with different fluorescently labeled probes; wherein said different fluorescently labeled probes have different wavelengths;
2) Cultivating said mutagenic seeds to obtain the first generation of mutation M1; self-cross of the first generation of mutation M1 is carried out to obtain the second generation of mutation M2;
3) Extracting genomic DNA of individual plant of the second generation of mutation M2; mixing genomic DNA of individual plant (with number n) of M2 to obtain a DNA pool; wherein n is 2-8;
4) Using each of said DNA pool as a template and both said primer pair B and fluorescently labeled primer pair B to obtain PCR products;
5) Digesting said PCR product by endonuclease CELI to obtain enzyme-digested product of a DNA pool;
6) Detecting the enzyme-digested products of each said DNA pool by electrophoresis; wherein if enzyme-digested products of said DNA pool generates bright dots under all wavelengths of different fluorescently labeled probes, individual plant of M2 (with number n) of said DNA pool contain or may contain fertility-lowered mutants or sterile mutants; wherein if enzyme-digested products of said DNA pool does not generate bright dots under all wavelengths of different fluorescently labeled probes, individual plants of M2 (with number n) of said DNA pool do not contain or may not contain fertility-lowered mutants or sterile mutants; wherein said fertility-lowered mutants are plants whose fertility is lower than that of said target plant.

**9.** The method according to claim 8, wherein following said step 6), said method further comprises the following steps:

genomic DNAs of individual plant (with number n) of M2 which contain or may contain fertility-lowered mutants or sterile mutants are mixed with genomic DNA of said target plant as templates; steps 4)-5) are repeated to obtain enzyme-digested products of individual plant of M2;
wherein each of said enzyme-digested products of individual plant of M2 is detected by electrophoresis; wherein if said enzyme-digested products of individual plant of M2 generate bright dots under all wavelengths of different fluorescently labeled probes, said individual plant of M2 is or may be a sterile mutant or fertility-lowered mutant; wherein if said enzyme-digested products of individual plant of M2 do not generate bright dots under all wavelengths of different fluorescently labeled probes, said individual plant of M2 is not or may not be a sterile mutant or fertility-lowered mutant.

**10.** The method according to claim 8 or 9,
wherein in step 1), treating a number of seeds of the target plant with sodium azide is immersing a number of seeds of the target plant in an aqueous solution of sodium azide with a concentration of 2mM for 6 hours;
wherein the amino acid sequence of said triterpene synthase is shown in SEQ ID NO. 2;
wherein said different fluorescently labeled probes are fluorescently labeled probe DY-682 with a wavelength of 682nm and fluorescently labeled probe DY-782 with a wavelength of 782nm;
wherein the nucleotide sequence of the gene encoding said triterpene synthase is shown in SEQ ID NO. 5;
wherein said primer pair B is selected from the following group consisting of primer pairs as shown in 1)-3):

1) Primer pair consisting of the single chain DNA molecule as shown in SEQ ID NO. 6 and the single chain DNA molecule as shown in SEQ ID NO. 7;
2) Primer pair consisting of the single chain DNA molecule as shown in SEQ ID NO. 8 and the single chain DNA molecule as shown in SEQ ID NO. 9;
3) Primer pair consisting of the single chain DNA molecule as shown in SEQ ID NO. 10 and the single chain DNA molecule as shown inSEQ ID NO. 11;

wherein aid target plant is a monocot plant; said monocot is a monocot graminaceous plant; specifically, said monocot graminaceous plant is rice.

**11.** A sterile mutant or fertility-lowered mutant prepared by the method of any one of claims 8 to 10.

**12.** The fertility-lowered mutant according to claim 11, wherein the Deposit Number of said fertility-lowered mutant is CGMCC NO.6150.

**13.** Use of a transgenic plant of claim 7 or said sterile mutant or fertility-lowered mutant of claim 11 or said fertility-lowered mutant of claim 12 in the production of hybrid seeds.

**14.** A method for obtaining a sterile mutant or fertility-lowered mutant, comprising silencing or inactivating the gene encoding triterpene synthase in a target plant; wherein said fertility-lowered mutant is a plant whose fertility is lower than that of the target plant.

**15.** The method according to claim 14, wherein said target plant is a monocot plant or dicot plant; said monocot plant is specifically a monocot graminaceous plant; said monocot graminaceous plant is rice, wheat, barley, sorghum or maize;
wherein the amino acid sequence of the triterpene synthase of said rice is shown in SEQ ID NO. 2; the nucleotide sequence of the gene encoding triterpene synthase of said rice is shown in SEQ ID NO. 5;
wherein the amino acid sequence of the triterpene synthase of said wheat is shown in SEQ ID NO. 15; the nucleotide sequence of the gene encoding triterpene synthase of said wheat is shown in SEQ ID NO. 14;
wherein the amino acid sequence of the triterpene synthase of said barley is shown in SEQ ID NO. 17; the nucleotide sequence of the gene encoding triterpene synthase of said barley is shown in SEQ ID NO. 16;
wherein the amino acid sequence of the triterpene synthase of said sorghum is shown in SEQ ID NO. 18; the nucleotide sequence of the gene encoding triterpene synthase of said sorghum is shown in SEQ ID NO. 19;
wherein the amino acid sequence of the triterpene synthase of said maize is shown in SEQ ID NO. 20; the nucleotide sequence of the gene encoding triterpene synthase of said maize is shown in SEQ ID NO. 21.

**16.** The method according to claim 14 or 15, wherein said silencing or inactivating of the gene encoding triterpene synthase in rice is at least one of the following 1)-3):

1) The nucleotide residue at the 764 position of said gene encoding triterpene synthase in rice is mutated from G to A;
2) The amino acid residue at the 809 position of said gene encoding triterpene synthase in rice is mutated from G to A;
3) The nucleotide residue at the 1431 position of said gene encoding triterpene synthase in rice is mutated from G to A.

**17.** A method for restoring or improving fertility of an original plant, comprising the following step: Maintaining the humidity for growth of plant inflorescence at 80-100% during anthesis of an original plant; said original plant is a sterile mutant or fertility-lowered mutant.

**18.** The method according to claim 17, wherein said sterile mutant or fertility-lowered mutant is said transgenic plant of claim 7 or said sterile mutant or fertility-lowered mutant of claim 11 or said fertility-lowered mutant of claim 12.

**19.** The method according to claim 17 or 18, wherein the time period of maintaining the humidity for growth of plant inflorescence is one week.

**20.** The method according to any one of claims 17 to 19, wherein the method of maintaining the humidity for growth of plant inflorescence is wrapping the whole inflorescence of said original plant;
wherein said wrapping is specifically using a plastic bag to slip over the whole inflorescence or using preservative film to cover the whole inflorescence.

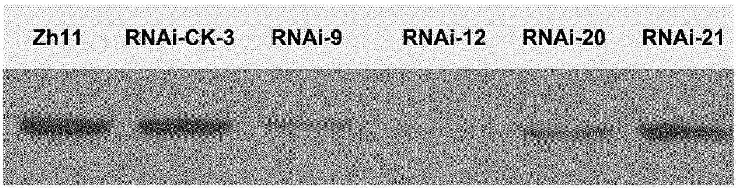


Figure 1

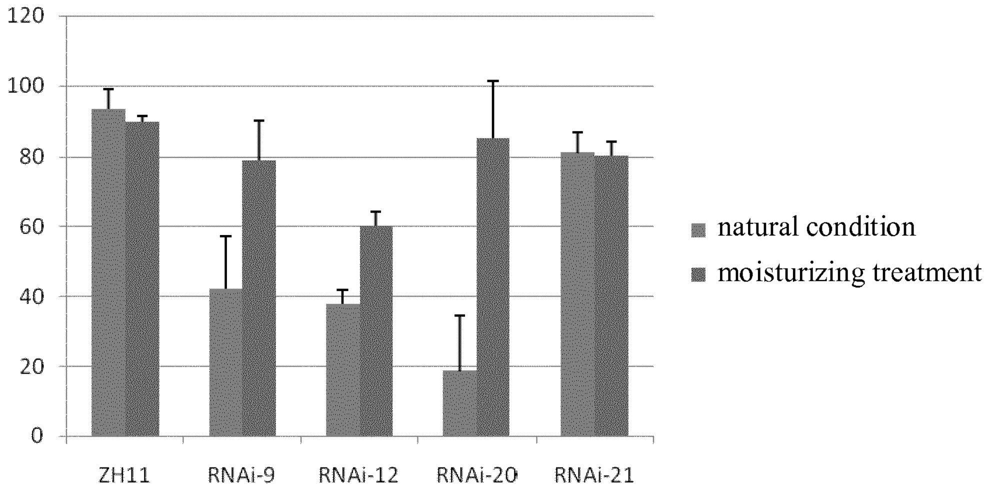


Figure 2

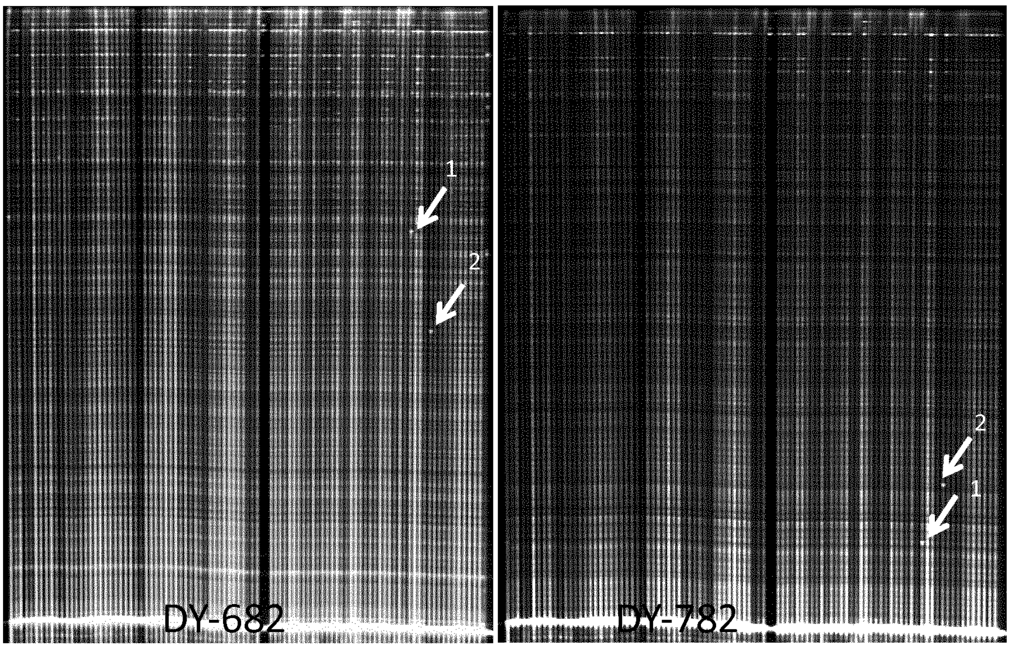


Figure 3

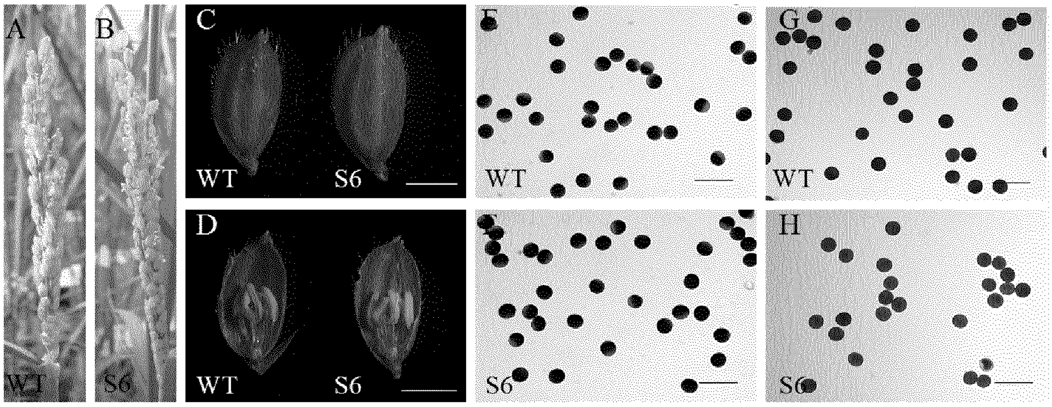


Figure 4

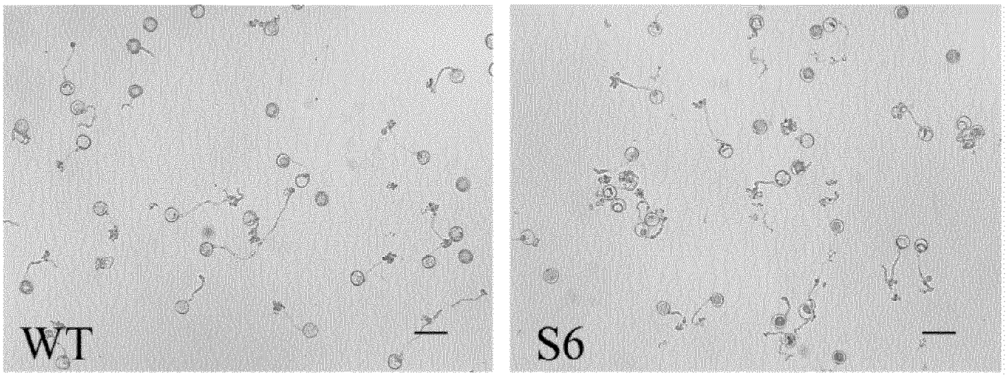


Figure 5

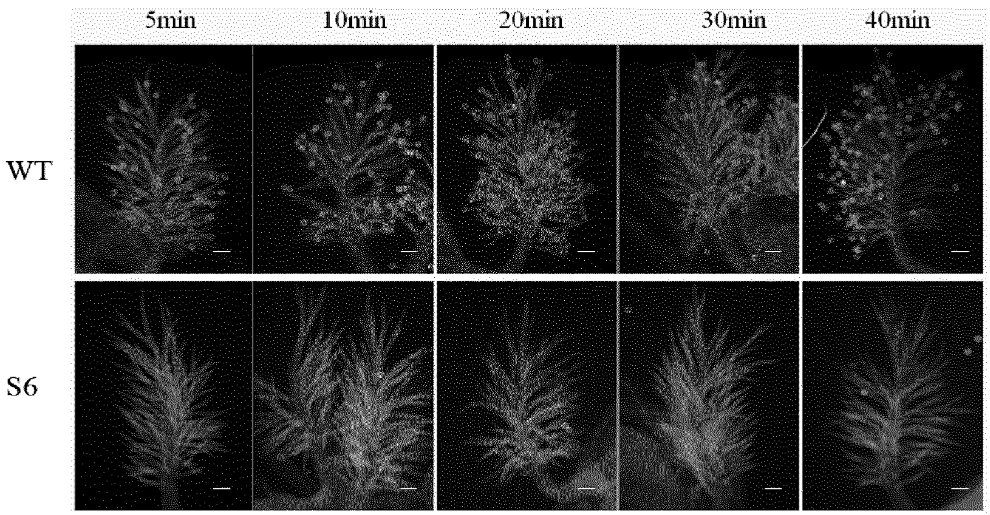

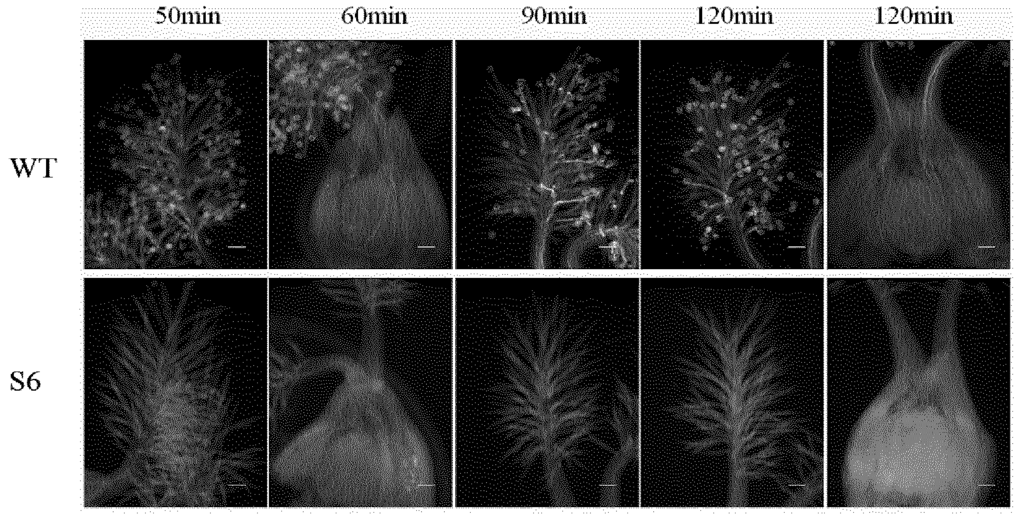


Figure 6

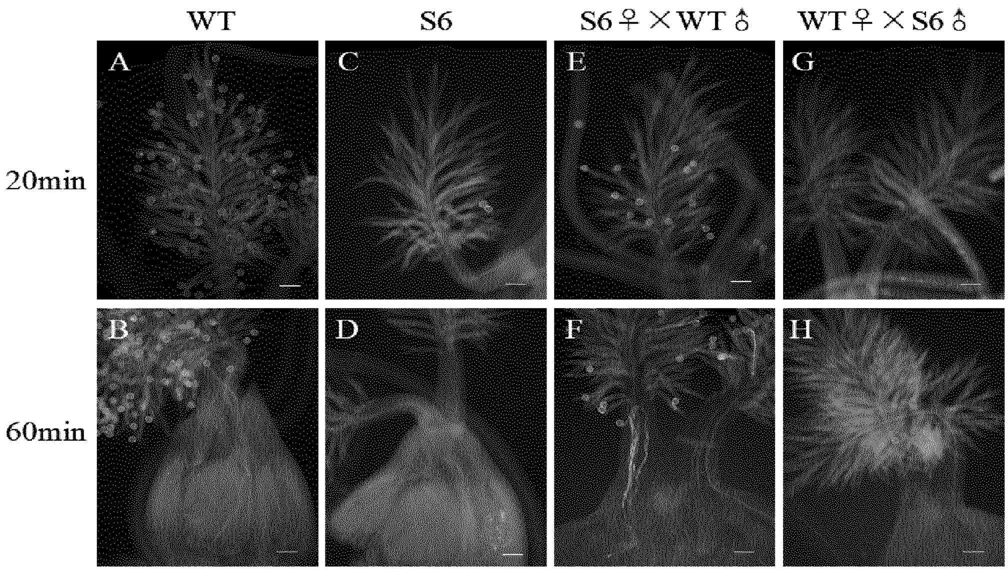


Figure 7

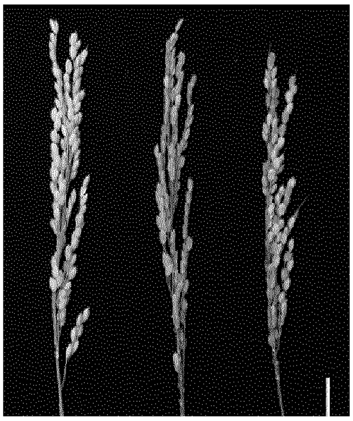

WT S6 S6 (moisturizing treatment)

Figure 8

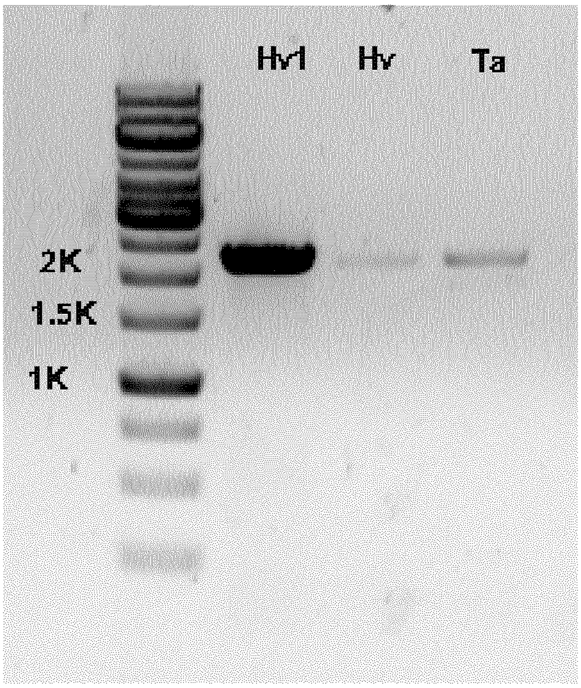


Figure 9

```
SbOSC1.txt   MWRLKIAKGGPWLKSGNSHIGRETWEFDQD.FGSKEEREAVDSAREEFKKNRFQMRHSSDILARMQLAKENGFSLDLQKT    79
OsOSC8.txt   MWKLKIAEGGPWLKSGNSHVGRETWEFDPN.FGTSEEREAVEAARIEFQKNRFRTRHTSDVLARMQLAKANNFSIDLQKE    79
HvOSC1.txt   MWKLKIAEGGPWLKSGNSHVGRETWEFDPN.FGTSEEREAVEAARIEFQKNRFRTRHTSDVLARMQLAKANNFSIDLQKE    79
TaOSC1.txt   MWKLKIAEGGPWLTSGNNHFGRETWEFDRNDAGSSEERDAVDAARAEFQKNRFRTRHSSDVLARMQLVKGNNFSLDQQQK    80
ZmOSC1.txt   MWRLKIGEGGPWLKSGNGHIGRETWEFDED.FGSEADREAVDSAREEFSKNRLRMRHSSDLLARMQIAKENGFSLDLHKT    79
Consensus

SbOSC1.txt   KDE...SPLVIN...SSTVSEILRKALNYFSAIQAHDGHWPGDFPGPLFTTATMIIVLYVTESLSSTLSSEHCKEICRYL   153
OsOSC8.txt   KDG...NPINID...TATVSDILKKALSYFSAIQAYDGHWPGDFPGPLFTTATMIIVLYVTESLTITLSSEHHKEICRYL   153
HvOSC1.txt   KDG...NPINID...TATVSDILKKALSYFSAIQAYDGHWPGDFPGPLFTTATMIIVLYVTESLTITLSSEHHKEICRYL   153
TaOSC1.txt   PKGD.ETSVDIN...IATVSETLKRALRYFSAIQAHDGHWPGDFPGPLFTTATMIVVLYVTESLGTTLSSEHRKEICRYL   156
ZmOSC1.txt   RDDDGRSPLLVATTGSSTVSETLRKALDYFAAIQAHDGHWPGDFPGPLFTTATMITVLYVTGSLDSTLSSEHRREICRYL   159
Consensus

SbOSC1.txt   YNRQNMDGGWGLHAEGESSMLSTALNYTTLRLLGENIDDG..PDMS....MLKARKWIHDHGGATMIPILGKVWLSVLGV   227
OsOSC8.txt   YNRQNIDGGWGLHAEGESSMLSTALNYTALRLLGENVDDG..PDIS....MHKARKWIHDHGGATMIPILGKVWLSVLGV   227
HvOSC1.txt   YNRQNIDGGWGLHAEGESSMLSTALNYTALRLLGENVDDG..PDIS....MHKARKWIHDHGGATMIPILGKVWLSVLGV   227
TaOSC1.txt   YNRQNMDGGWGLHAEGESSMLSSALNYAALRLLGEGADDG..PDMS....MPKARKWIHDHGGATMIPILGKVWLSVLGV   230
ZmOSC1.txt   HNRQNADGGWGLHAEGESSMLSTALNYTALRLLGEGGDGGGGPGMSSSSMLIRARKWIRDRGGATMIPILGKVWLSVLGV   239
Consensus

SbOSC1.txt   FEWSGVNPIPPELFLLPSWVPIQPGRLWSHFRMAFIPMCYLYGKKFVGPITRLVMSLREELHIHPYKKIDWKQARKLCAK   307
OsOSC8.txt   FDWSGVNPIPPELFLLPSFVPIQPGRLWSHFRMAFIPMSYLYGKKFVGPITRLVISLREELHIHPYKKIDWKEARKLCAK   307
HvOSC1.txt   FDWSGVNPIPPELFLLPSFVPIQPGRLWSHFRMAFIPMSYLYGKKFVGPITRLVISLREELHIHPYKKIDWKEARKLCAK   307
TaOSC1.txt   SEWSGVNPMPPELFLLPSFVPIQPGRLWSHFRMAFIPMSYLYGKKFVGPITKLVLSLREELHIHPYKKINWKQARKLCAK   310
ZmOSC1.txt   FEWSGVNPIPPELFLFPSWVPIQPGRLWSHFRMAFIPMSYLYGKKFVGPITRLVVSLREELHVHPYEKIDWKAARNSCAK   319
Consensus

SbOSC1.txt   EDVYNPHTWLQECLSDCLYSFGEPFLTRWPISYMRKKALKQVAEFLKYEDENSQYICIGAAQKALSMLCCWIEKSNSDAF   387
OsOSC8.txt   EDAYNPHMWLQECLSDCLYSFGEPFLTRWPISYMRKRALYQIAEFLKYEDENSQYICIGAAQKALSMLCCWIENPNSDAF   387
HvOSC1.txt   EDAYNPHMWLQECLSDCLYSFGEPFLTRWPISYMRKRALYQIAEFLKYEDENSQYICIGAAQKALSMLCCWIENPNSDAF   387
TaOSC1.txt   EDAYHPHTWLQECLSDCLYSFGEPFLACWPVSHMRRKALRQIADFLKYEDDISRYICIGAAQKALSMLCCWSENPSSDAF   390
ZmOSC1.txt   EDVYSPHTWLQECLSHCLYSFGEPFLTRWPISYMRKKALQQVAEFLKYEDENSQYICIGAAQKALSMLCCWIEKSNSDAF   399
Consensus

SbOSC1.txt   KRHLARVADFLWIGEDGMKVRVCAGQLWDVAFAVQAILACNIAEEYRSTLKKAHDFIKASQIMDNPSGDFSRKYRHISKG   467
OsOSC8.txt   KRHLARVADFLWVGEDGMKVRVCAGQLWDVAFAVQAILACSIAEEFGSTLKKAHGFIKTSQIMDNPSGDFSRKYRHISKG   467
HvOSC1.txt   KRHLARVADFLWVGEDGMKVRVCAGQLWDVAFAVQAILACSIAEEFGSTLKKAHGFIKTSQIMDNPSGDFSRKYRHISKG   467
TaOSC1.txt   KRHLARVSDFLWLGEDGMKVRVCAGQSWDVAFAVQAILACDVAQEFGTTLKKAHHFIKASQIVSNPTGDFSRKYRHISKG   470
ZmOSC1.txt   KRHLARVADFLWIGEDGMKVRVCAGQLWDVAFAVQAILACNIAEEYRGTLKKAHDFIKASQIMDNPSGDFSRKYRHISKG   479
Consensus

SbOSC1.txt   GWGFQVADQGWQVSDCTAEALKVLLMLSKFSSDIGSDQMETCRLYNAVNVLLSLQNPNGGYGTWELARTYPWMEIFNMTE   547
OsOSC8.txt   GWAFQVADQGWQVSDCTAEALKALLLLSKCLSDGADYQMETYCYFDAVNVLLSLQNPNGGYGAWELARTYPWMEIFNMTE   547
HvOSC1.txt   GWAFQVADQGWQVSDCTAEALKALLLLSKCLSDGADYQMETYCYFDAVNVLLSLQNPNGGYGAWELARTYPWMEIFNMTE   547
TaOSC1.txt   GWAFQVADQGWQVSDCTAEALKALLLLSKFPSEIVGDQMETCRFHDAVNILLSLQNPNGGYGTWELARTYPWMENLNMTE   550
ZmOSC1.txt   GWGFQVADQGWQVSDCTAEALKVLLMLSRFSSDIGSDQMETCRLYDAVNVLLSLQNPNGGYGTWELARTYPWIEIFNMTE   559
Consensus

SbOSC1.txt   IYADIMVEHQYVECTSSVMQALALFQEKYPWHRKDEIDQCIRGATEFIEKLQNDDGSWFGSWGICFTYGTWFAIEGLSAV   627
OsOSC8.txt   IYADIIVEHQYVECTSSVIQALALFREKYPGHRKDEIDQCIRKATEFIEKLQNDDGSWFGSWGICFTYGTWFAIEGLSAV   627
HvOSC1.txt   IYADIIVEHQYVECTSSVIQALALFREKYPGHRKDEIDQCIRKATEFIEKLQNDDGSWFGSWGICFTYGTWFAIEGLSAV   627
TaOSC1.txt   IYADIMVEHQYVECTSSVIQALALFRQKYPGHREDEVEQCIRRATEFIEKLQNEDGSWFGSWGICFTYGTWFAIEGLSAV   630
ZmOSC1.txt   IYADIMVEHQYVECTSSVMQALVLFREKDPWHRKDEIDQCIRGATEFIEKLQNDDGSWFGSWGICFTYGTWFAIEGLSAV   639
Consensus

SbOSC1.txt   GQSYGNSTCIRKACKFLLTKQLNNGGWGESYLSSRTKAYTNLDRQKSHIVNTAWAMLALMKAGQVERDPTPLHKAARLIM   707
OsOSC8.txt   GQCYDDSTCIRKACKFLLSKQLTNGGWGESHLSSRTKAYTNLDGEKSHIVNTAWAMLALMKAGQVERDPAPLHKAARLIM   707
HvOSC1.txt   GQCYDDSTCIRKACKFLLSKQLTNGGWGESHLSSRTKAYTNLDGEKSHIVNTAWAMLALMKAGQVERDPAPLHKAARLIM   707
TaOSC1.txt   GQCYNNSTYIRKGCQFLLSKQLRNGGWGESHLSSTTKAYTNLDGEKSHVVNTAWAMLALMKAGQAERDPSPLHEAARLIM   710
ZmOSC1.txt   GQSYGNSTCIQKACKFLLAKQLKNGGWGESHLSSTTKAYTNLDKEKSHIVNTAWAMLALMKAGQAERDPTPLHKAARLIM   719
Consensus

SbOSC1.txt   SMQLGNGDFPQEEMIGSFLKNGPLCYMAYRNIFPIWALGEYQKLVLQ....CDLQRPTF.................   762
OsOSC8.txt   SMQLSDGDFPQEEMIGSFLKNGPLCYMAYRNIFPIWALGEYQKLVFQNYQTSSIKQTNIAPSAGNAALKNSASTTAP   784
HvOSC1.txt   SMQLSDGDFPQEEMIGSFLKNGPLCYMAYRNIFPIWALGEYQKLVFQNYQTSSIKQTNIAPSAGNAALKNSASTTAP   784
TaOSC1.txt   SMQLGNGDFPQEEMIGSFLKNGPLCYMAYRNIFPIWALGEYHRLVLCSG............................   759
ZmOSC1.txt   SMQLSNGDFPQEEMIGSFLKNGPLCYMAYRNIFPIWALGEYQKLVLQ....CDLQWPTF.................   774
Consensus
```

Figure 10

# INTERNATIONAL SEARCH REPORT

International application No.
**PCT/CN2012/001546**

**A. CLASSIFICATION OF SUBJECT MATTER**

See the extra sheet

According to International Patent Classification (IPC) or to both national classification and IPC

**B. FIELDS SEARCHED**

Minimum documentation searched (classification system followed by classification symbols)

IPC: C12N A01H

Documentation searched other than minimum documentation to the extent that such documents are included in the fields searched

Electronic data base consulted during the international search (name of data base and, where practicable, search terms used)

Data bases: CPEA, DWPI, JPABS, SIPOABS, VEN, CPRSABS, MOABS, CNABS, TWABS, CJFD, CSCD, SIPONPL USTXT, JPTXT, EPTXT, WOTXT, cnki, isi web of knowledge, GenBank+EMBL+DDBJ

Search terms: triterpene synthases, improvement, Terpenoid Synthases sterile, oxidosqualene cyclase, oxidosqualene synthase (OSC), pollen, stigma, humidity, inflorescence, fertility, sterile, revert, restore, tilling or (targeting w induced w local w lesions); search on SEQ ID NO: 1, 2, 5 and 14-21

**C. DOCUMENTS CONSIDERED TO BE RELEVANT**

| Category* | Citation of document, with indication, where appropriate, of the relevant passages | Relevant to claim No. |
|---|---|---|
| X | PREUSS, D. et al., "A conditional sterile mutation eliminates surface components from Arabidopsis pollen and disrupts cell signaling during fertilization", GENES & DEVELOPMENT, ISSN 0890-9369/93, no. 7, pages 974-985, 31 December 1993 (31.12.1993), see pages 977-978, figure 4 | 17, 19-20 |
| A | | 1, 16, 18 |
| A | EP 2339008 A1 (CRA CONSIGLIO RICERCA E SPERIMENTAZIONE IN AGRICOLTURA et al.), 29 June 2011 (29.06.2011), see the whole document | 1-20 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 31 January 2013 (31.01.2013) | **14 February 2013 (14.02.2013)** |

| Name and mailing address of the ISA/CN | Authorized officer |
|---|---|
| State Intellectual Property Office of the P. R. China<br>No. 6, Xitucheng Road, Jimenqiao<br>Haidian District, Beijing 100088, China<br>Facsimile No.: (86-10) 62019451 | **WANG, Qiyang**<br>Telephone No.: (86-10) **62411091** |

Form PCT/ISA/210 (second sheet) (July 2009)

# INTERNATIONAL SEARCH REPORT

International application No. PCT/CN2012/001546

| C (Continuation). DOCUMENTS CONSIDERED TO BE RELEVANT | | |
|---|---|---|
| Category* | Citation of document, with indication, where appropriate, of the relevant passages | Relevant to claim No. |
| A | US 2009044289 A1 (AGRIC VICTORIA SERVICES PTY LTD. et al.), 12 February 2009 (12.02.2009), see the whole document | 1-20 |
| A | ZHANG, Changbo et al., "Plant Terpenoid Natural Metabolism Pathways and Their Synthases", PLANT PHYSIOLOGY COMMUNICATIONS, ISSN 2095-1108, vol. 43, no. 4, pages 779-784, 31 August 2007 (31.08.2007), see the whole document | 1-20 |
| A | SUN, Jie et al., "TILLING Technology and Its Application", CHINESE JOURNAL OF CELL BIOLOGY, ISSN 1674-7666, no. 29, pages 41-46, 31 December 2007 (31.12.2007), see the whole document | 1-20 |

Form PCT/ISA/210 (continuation of second sheet) (July 2009)

# INTERNATIONAL SEARCH REPORT

International application No.
**PCT/CN2012/001546**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See the extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

# INTERNATIONAL SEARCH REPORT

International application No.
**PCT/CN2012/001546**

**CONTINUATION OF BOX A:**

IPC

C12N 15/63 (2006.01) i

C12N 15/52 (2006.01) i

C12N 9/00 (2006.01) i

A01H 1/00 (2006.01) i

A01H 5/00 (2006.01) i

**Continuation of Box No. III**

Claims 1-20 can be divided into the following two groups of inventions

Group 1: claims 1-16 and 18-20

Group 2: claims 17 and 18-20

The common special technical feature involved in claims of group 1 is: obtaining fertility decreased or sterile plants by inactivating triterpene synthase genes of plants; and the common special technical feature involved in claims of group 2 is: recovering the fertility of plants by maintaining the growth humidity of inflorescence at 80-10%. However, claim 17 and claims 1-16 do not share a same or corresponding special technical feature, and do not belong to a technical concept, therefore do not meet the requirement of PCT Rule 17.(3)(a).

Form PCT/ISA/210 (extra sheet) (July 2009)

# INTERNATIONAL SEARCH REPORT
Information on patent family members

International application No.
**PCT/CN2012/001546**

| Patent Documents referred in the Report | Publication Date | Patent Family | Publication Date |
|---|---|---|---|
| EP 2339008 A1 | 29.06.2011 | None | |
| US 2009044289 A1 | 12.02.2009 | US 20120185963 A1 | 19.07.2012 |
| | | IN 200700310 P1P1 | 17.08.2007 |
| | | US 8163975 B2 | 24.04.2012 |
| | | WO 2005122751 A1 | 29.12.2005 |
| | | AU 2005253642 BB2 | 09.06.2011 |
| | | EP 1765058 A1 | 28.03.2007 |
| | | AU 2005253642 A2 | 29.12.2005 |
| | | ZA 200700456 A | 25.11.2009 |
| | | EP 2241624 A2 | 20.10.2010 |
| | | NZ 552613 A | 30.10.2009 |
| | | EP 2241624 A3 | 09.02.2011 |
| | | AU 2005253642 BB8 | 19.01.2012 |
| | | AU 2005253642 A1 | 29.12.2005 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PREUSS et al.** *Gene Dev.,* 1993, vol. 7, 947-985
- **ZHONG-HAI REN et al.** A rice quantitative trait locus for salt tolerance encodes a sodium transporter. *Nature Genetics,* 2005, vol. 37, 1141-1146
- **DAMME et al.** Somatic Cytokinesis and Pollen Maturation in Arabidopsis Depend on TPLATE, Which Has Domains Similar to Coat Proteins. *plant cell,* 2006, vol. 18, 3502-3518
- **K L PIERS et al.** Agrobacterium tumefaciens-mediated transformation of yeast. *PNAS,* 20 February 1996, vol. 93 (4), 1613-1618
- **ALEXANDER MP.** *Stain Technol,* 1969, vol. 44, 117-122
- **ENDO M et al.** *Plant Cell Physiol,* 2009, vol. 50, 1911-1922
- **JUN YU ; SONGNIAN HU ; JUN WANG ; GANE KA-SHU WONG ; JIAN WANG ; LIHUANG ZHU ; LONGPING YUAN ; HUANMING YANG.** A draft sequence of the rice (Oryza sativa ssp. indica) genome. *Science,* 2002, vol. 296, 79-92
- **L. JIZENG JIA ; ZHENGBIN ZHANG ; K. DEVOS ; M. D. GALE.** Analysis of genetic diversity of 21 chromosomes of Triticum aestivum L. based on RFLP mapping sites. *Science in China Series C: Life Sciences,* 2001
- **L. QI X ; NIKS RE ; STAM P ; LINDHOUT P.** Identification of QTLs for partial resistance to leaf rust (Puccinia hordei) in barley. *Theor Appl Genet,* 1998, vol. 96, 1205-1215

<110> INSTITUTE OF BOTANY, CHINESE ACADEMY OF SCIENCE

<120> METHOD FOR PREPARING FERTILITY-LOWERED PLANT

<160> 21

<210> 1
<211> 201
<212> DNA
<213> Synthetic
<220>
<223>

<400> 1
ggctgcacgg atagagttcc agaagaatag gttcagaaca agacacacct ccgatgtttt 60

ggctcgcatg cagttagcta aggcgaacaa cttcagtatt gatctacaga aagaaaaaga 120

tggaaacccc ataaatattg acacagctac agtatcagat atactgaaga aggcactcag 180

ttatttctca gccatacagg c 201

<210> 2
<211>785
<212> PRT
<213> rice Oryza sativa
<400> 2
Met Trp Lys Leu Lys Ile Ala Glu Gly Gly Pro Trp Leu Lys Ser Gly
1 5 10 15

Asn Ser His Val Gly Arg Glu Thr Trp Glu Phe Asp Pro Asn Phe Gly
20 25 30

Thr Ser Glu Glu Arg Glu Ala Val Glu Ala Ala Arg Ile Glu Phe Gln
35 40 45

Lys Asn Arg Phe Arg Thr Arg His Thr Ser Asp Val Leu Ala Arg Met
50 55 60

Gln Leu Ala Lys Ala Asn Asn Phe Ser Ile Asp Leu Gln Lys Glu Lys
65 70 75 80

Asp Gly Asn Pro Ile Asn Ile Asp Thr Ala Thr Val Ser Asp Ile Leu
85 90 95

Lys Lys Ala Leu Ser Tyr Phe Ser Ala Ile Gln Ala Tyr Asp Gly His
100 105 110

Trp Pro Gly Asp Phe Pro Gly Pro Leu Phe Thr Thr Ala Thr Met Ile
115 120 125

Ile Val Leu Tyr Val Thr Glu Ser Leu Thr Ile Thr Leu Ser Ser Glu
130 135 140

His His Lys Glu Ile Cys Arg Tyr Leu Tyr Asn Arg Gln Asn Ile Asp
145 150 155 160

```
Gly Gly Trp Gly Leu His Ala Glu Gly Glu Ser Ser Met Leu Ser Thr
                165                 170                 175

Ala Leu Asn Tyr Thr Ala Leu Arg Leu Leu Gly Glu Asn Val Asp Asp
            180                 185                 190

Gly Pro Asp Ile Ser Met His Lys Ala Arg Lys Trp Ile His Asp His
        195                 200                 205

Gly Gly Ala Thr Met Ile Pro Ile Leu Gly Lys Val Trp Leu Ser Val
    210                 215                 220

Leu Gly Val Phe Asp Trp Ser Gly Val Asn Pro Ile Pro Pro Glu Leu
225                 230                 235                 240

Phe Leu Leu Pro Ser Phe Val Pro Ile Gln Pro Gly Arg Leu Trp Ser
                245                 250                 255

His Phe Arg Met Ala Phe Ile Pro Met Ser Tyr Leu Tyr Gly Lys Lys
            260                 265                 270

Phe Val Gly Pro Ile Thr Arg Leu Val Ile Ser Leu Arg Glu Glu Leu
        275                 280                 285

His Ile His Pro Tyr Lys Lys Ile Asp Trp Lys Glu Ala Arg Lys Leu
    290                 295                 300

Cys Ala Lys Glu Asp Ala Tyr Asn Pro His Met Trp Leu Gln Glu Cys
305                 310                 315                 320

Leu Ser Asp Cys Leu Tyr Ser Phe Gly Glu Pro Phe Leu Thr Arg Trp
                325                 330                 335

Pro Ile Ser Tyr Met Arg Lys Arg Ala Leu Tyr Gln Ile Ala Glu Phe
            340                 345                 350

Leu Lys Tyr Glu Asp Glu Asn Ser Gln Tyr Ile Cys Ile Gly Ala Ala
        355                 360                 365

Gln Lys Ala Leu Ser Met Leu Cys Cys Trp Ile Glu Asn Pro Asn Ser
    370                 375                 380

Asp Ala Phe Lys Arg His Leu Ala Arg Val Ala Asp Phe Leu Trp Val
385                 390                 395                 400

Gly Glu Asp Gly Met Lys Val Arg Val Cys Ala Gly Gln Leu Trp Asp
                405                 410                 415

Val Ala Phe Ala Val Gln Ala Ile Leu Ala Cys Ser Ile Ala Glu Glu
            420                 425                 430
```

Phe Gly Ser Thr Leu Lys Lys Ala His Gly Phe Ile Lys Thr Ser Gln
435 440 445

Ile Met Asp Asn Pro Ser Gly Asp Phe Ser Arg Lys Tyr Arg His Ile
450 455 460

Ser Lys Gly Gly Trp Ala Phe Gln Val Ala Asp Gln Gly Trp Gln Val
465 470 475 480

Ser Asp Cys Thr Ala Glu Ala Leu Lys Ala Leu Leu Leu Leu Ser Lys
485 490 495

Cys Leu Ser Asp Gly Ala Asp Tyr Gln Met Glu Thr Tyr Cys Tyr Phe
500 505 510

Asp Ala Val Asn Val Leu Leu Ser Leu Gln Asn Pro Asn Gly Gly Tyr
515 520 525

Gly Ala Trp Glu Leu Ala Arg Thr Tyr Pro Trp Met Glu Ile Phe Asn
530 535 540

Met Thr Glu Ile Tyr Ala Asp Ile Ile Val Glu His Gln Tyr Val Glu
545 550 555 560

Cys Thr Ser Ser Val Ile Gln Ala Leu Ala Leu Phe Arg Glu Lys Tyr
565 570 575

Pro Gly His Arg Lys Asp Glu Ile Asp Gln Cys Ile Arg Lys Ala Thr
580 585 590

Glu Phe Ile Glu Lys Leu Gln Asn Asp Asp Gly Ser Trp Phe Gly Ser
595 600 605

Trp Gly Ile Cys Phe Thr Tyr Gly Thr Trp Phe Ala Ile Glu Gly Leu
610 615 620

Ser Ala Val Gly Gln Cys Tyr Asp Asp Ser Thr Cys Ile Arg Lys Ala
625 630 635 640

Cys Lys Phe Leu Leu Ser Lys Gln Leu Thr Asn Gly Gly Trp Gly Glu
645 650 655

Ser His Leu Ser Ser Arg Thr Lys Ala Tyr Thr Asn Leu Asp Gly Glu
660 665 670

Lys Ser His Ile Val Asn Thr Ala Trp Ala Met Leu Ala Leu Met Lys
675 680 685

Ala Gly Gln Val Glu Arg Asp Pro Ala Pro Leu His Lys Ala Ala Arg
690 695 700

Leu Ile Met Ser Met Gln Leu Ser Asp Gly Asp Phe Pro Gln Glu Glu
705 710 715 720

Met Ile Gly Ser Phe Leu Lys Asn Gly Pro Leu Cys Tyr Met Ala Tyr
725 730 735

Arg Asn Ile Phe Pro Ile Trp Ala Leu Gly Glu Tyr Gln Lys Leu Val
740 745 750

Phe Gln Asn Tyr Gln Thr Ser Ser Ile Lys Gln Thr Asn Ile Ala Pro
755 760 765

Ser Ala Gly Asn Ala Ala Leu Lys Asn Ser Ala Ser Thr Thr Ala Pro
770 775 780

Thr
785

<210> 3
<211> 30
<212> DNA
<213> Synthetic
<220>
<223>

<400> 3
aaaaagcagg ctggctgcac ggatagagtt 30

<210> 4
<211> 30
<212> DNA
<213> Synthetic
<220>
<223>

<400> 4
agaaagctgg gtgcctgtat ggctgagaaa 30
<210> 5
<211> 2358
<212> DNA
<213> rice Oryza sativa

<400> 5
atgtggaagc tcaagattgc cgagggagga ccatggctaa agagtggaaa cagtcatgtt 60
ggaagagaaa catgggaatt tgacccaaat tttggaacaa gtgaagagcg ggaggcggtt 120
gaggctgcac ggatagagtt ccagaagaat aggttcagaa caagacacac ctccgatgtt 180
ttggctcgca tgcagttagc taaggcgaac aacttcagta ttgatctaca gaaagaaaaa 240
gatggaaacc ccataaatat tgacacagct acagtatcag atatactgaa gaaggcactc 300
agttatttct cagccataca ggcatatgat gggcactggc caggggattt cccaggccca 360
ctgtttacca ctgcaaccat gatcatagtt ctatatgtca cagagtcatt aactattaca 420
ctgtcatcgg aacatcacaa ggagatctgt cgatatctgt acaatcgtca gaacatcgat 480
ggaggatggg gactacatgc agaaggtgaa agttccatgc ttagcacagc tctcaattat 540
actgctctga gactacttgg ggagaatgtt gatgatggac cagatatatc catgcataaa 600
gcaagaaaat ggatacatga ccatggaggt gcaacaatga taccgatctt gggaaaagtg 660
tggctctcgg tgcttggagt ttttgactgg tcaggtgtaa atcctattcc cccagaatta 720
tttcttcttc catctttcgt tcctatccaa ccaggacgat tatggagtca cttccgaatg 780
gcttttattc ccatgtctta tttgtatgga aaaaagtttg tcggtcctat aacaagattg 840
gttatatcat taagggaaga gctacatatt catccctata aaaagattga ctggaaggag 900
gcacgcaaat tatgtgcaaa ggaagatgcc tataatccac atatgtggct acaagagtgc 960
ctatctgact gcctttatag ttttggtgag ccgtttctta cacgttggcc aatttcctac 1020
atgagaaaaa gagctctata ccaaattgct gagttcttga agtatgaaga tgaaaattct 1080
cagtatatct gcattggtgc tgcacagaag gcattatcca tgttgtgctg ttggattgaa 1140

```
aatcccaatt cagatgcatt caagcgtcat ttggctagag tagctgattt tctatgggtt    1200
ggcgaagatg gcatgaaagt tcgggtctgt gcgggtcaat tatgggatgt tgctttcgca    1260
gtccaagcga tattagcgtg tagcatcgca gaagaatttg gaagtaccct taagaaagca    1320
catggtttca taaaaacttc ccagattatg gacaatcctt ctggtgactt cagcagaaag    1380
taccgtcaca tatctaaagg aggatgggcc ttccaggttg cagatcaggg gtggcaggtt    1440
tcagattgca cagcagaagc tcttaaggct ttgctactgc tgtcaaagtg tttgtcagat    1500
ggtgcagatt atcaaatgga aacctactgc tactttgacg cggtgaatgt attactctcg    1560
ttacagaacc caaatggtgg gtatggagca tgggagctag ctcgcacata tccatggatg    1620
gagattttca acatgacaga gatatatgca gacatcattg tggagcatca gtacgtagag    1680
tgtacctcat cagtcatcca agcattggca ttgttccggg agaagtaccc tgggcatcgg    1740
aaagatgaaa tagatcaatg catcaggaaa gcaacagaat tcatcgagaa gttacagaat    1800
gatgatggat catggtttgg atcatggggt atttgcttca cgtatggaac atggtttgca    1860
atagagggtc tgtcagccgt ggggcagtgt tatgatgaca gcacctgcat tcggaaagcc    1920
tgtaagtttc tgttatcaaa gcaactaaca aatggtggat gggggggagag tcatctttca    1980
tccagaacca aggcatacac aaacctagat ggggagaaat cacatatagt caatactgca    2040
tgggcaatgt tggcactcat gaaagctgga caggtagaga gagacccccgc tcctctgcac    2100
aaagcagcaa gacttatcat gagcatgcag ctcagtgatg gtgactttcc acaagaggaa    2160
atgatcggaa gtttcttgaa aaatggcccc ttgtgttata tggcttatcg caatatattc    2220
ccaatatggg ctcttggaga gtatcagaaa ttagtatttc agaactatca gacctctagc    2280
atcaagcaaa caaatatagc tccatctgct ggcaatgctg ctctgaagaa ttcagcaagt    2340
acaacagcgc caacttga                                                  2358
```

<210> 6
<211> 25
<212> DNA
<213> Synthetic
<220>
<223>

<400> 6
gaggtcaagt cgtcttctgc aatta 25

<210> 7
<211> 22
<212> DNA
<213> Synthetic
<220>
<223>

<400> 7
atttgtctgc gctctgcaca tg 22

<210> 8
<211> 25
<212> DNA
<213> Synthetic
<220>
<223>

<400> 8
gcttaaaggt aaatttcagg cttcc 25

<210> 9
<211> 25
<212> DNA
<213> Synthetic
<220>
<223>

<400> 9
cgatcagaat caattaaacc cagac 25

<210> 10
<211> 25
<212> DNA
<213> Synthetic
<220>
<223>

<400> 10
tcatccttag attaattagc cgaca 25

<210> 11
<211> 25
<212> DNA
<213> ÈË¹¤ºÏ³É
<220>
<223>

<400> 11
cataaggatc tcataaaatc gacca 25
<210> 12
<211> 20
<212> DNA
<213> Synthetic
<220>
<223>

<400> 12
atgtggaagc tcaagatcgc 20
<210> 13
<211> 22
<212> DNA
<213> Synthetic
<220>
<223>

<400> 13
ttagccagag caaagtacta at 22

<210> 14
<211> 2280
<212> DNA
<213> wheat Triticum aestivum
<400> 14
atgtggaagc tcaagatcgc cgagggcggc ccgtggctca cgagcggcaa caaccacttc 60
ggaagagaaa catgggagtt tgaccgaaat gacgctggat caagcgaaga gcgggatgcg 120
gtcgacgctg cacgggctga attccagaag aacaggttca ggacaaggca cagctccgat 180
gttttggccc gcatgcagtt agtaaagggg aataacttca gccttgacca acaacagaaa 240
ccaaaaggtg atgaaactag tgtcgacatc aacatagcta cggtgtcgga gacactgaaa 300
agggcactca gatacttctc agccatacaa gcgcacgatg ggcactggcc aggagatttt 360
ccgggccctc tctttaccac agcaaccatg atcgtagttt tgtatgtcac ggagtcgtta 420
ggtactacgc tatcgtcaga acaccgcaag gagatctgtc gctatttgta caaccgacag 480
aatatggatg gaggatgggg actacatgcg gaaggcgaga gctccatgct cagctcagct 540
ctcaactacg ctgctctaag actacttggt gagggtgctg atgatggacc agatatgtcc 600
atgccaaaag ctaggaaatg gatacatgac catggtggtg caacaatgat accaatattg 660
ggaaaagtgt ggctttcggt acttggggtt tctgaatggt caggtgtaaa ccctatgccc 720
ccagaattgt tccttctgcc atccttcgtt cctatccaac caggacggtt gtggagtcac 780
ttcagaatgg ctttcatccc catgtcctat ttatatggca agaagtttgt tggcccaata 840
acaaaactgg ttttatcatt aagggaagag ctgcatattc acccctacaa aaagattaac 900
tggaagcaag cgcgcaaatt atgcgcaaag gaagatgcgt atcatccaca tacctggctc 960
caagaatgct tgtccgattg cctttacagt ttcggtgaac ctttttctggc atgttggcca 1020
gtttcccaca tgagacgaaa agctctacga caaattgccg atttcctgaa atacgaagat 1080
gatatttcac ggtatatctg cattggcgct gcgcaaaagg cattatccat gttatgctgt 1140
tggtctgaga atcccagttc agatgcattc aagcgccact tggctagagt ttctgatttc 1200
ctatggctcg gtgaagatgg catgaaagtg cgggtatgtg caggtcagtc atgggatgtt 1260
gcttttgctg tacaagcaat attagcgtgt gatgttgcac aggaatttgg aactactctg 1320
aagaaagcac accatttcat aaaagcatcc cagattgtca gcaatcctac cggtgacttc 1380
agcagaaagt accgtcacat ctctaaagga ggatgggcct tccaggttgc agaccagggc 1440
tggcaggttt cagactgcac agcagaagct ctcaaggctc tgttactgct ctcaaagttt 1500
ccgtcagaga tcgtgggtga tcagatggaa acatgccgct tccatgatgc agtgaacata 1560
ttattatctt tacagaatcc taatggtggc tatggaactt gggagctagc tcgtacatat 1620
ccatggatgg agaatttaaa catgacagag atatatgcag acatcatggt ggagcatcag 1680
tacgtcgagt gtacctcgtc ggtcatccaa gcattggccc tgtttcggca aaaatacccc 1740
gggcatcggg aagatgaagt agaacaatgc atcaggagag cgacagaatt catcgagaag 1800
ttacagaatg aggacggttc atggttcgga tcatggggta tttgcttcac atacggcacg 1860
tggtttgcta tagagggcct atcggcagtt ggacagtgtt acaataatag cacctacatc 1920
cggaagggtt gccagtttct attatcaaag cagctaagga atggtggatg gggtgagagt 1980
catctttcat ccacaaccaa ggcatacacg aacctagacg gggagaaatc acatgtagtc 2040
aacaccgcat gggcaatgtt ggcactaatg aaagctggac aggccgaacg agatccgtct 2100

cctttgcacg aagctgcaag acttatcatg agcatgcaac ttggcaatgg tgacttccca 2160
caggaggaaa tgattggaag tttcttgaaa aatggtccct tgtgttatat ggcttatcgc 2220
aacatattcc ccatatgggc ccttggagag tatcatagat tagtactttg ctctggctaa 2280

<210> 15
<211> 759
<212> PRT
<213> wheat Triticum aestivum
<400> 15

Met Trp Lys Leu Lys Ile Ala Glu Gly Gly Pro Trp Leu Thr Ser Gly
1 5 10 15

Asn Asn His Phe Gly Arg Glu Thr Trp Glu Phe Asp Arg Asn Asp Ala
20 25 30

Gly Ser Ser Glu Glu Arg Asp Ala Val Asp Ala Ala Arg Ala Glu Phe
35 40 45

Gln Lys Asn Arg Phe Arg Thr Arg His Ser Ser Asp Val Leu Ala Arg
50 55 60

Met Gln Leu Val Lys Gly Asn Asn Phe Ser Leu Asp Gln Gln Gln Lys
65 70 75 80

Pro Lys Gly Asp Glu Thr Ser Val Asp Ile Asn Ile Ala Thr Val Ser
85 90 95

Glu Thr Leu Lys Arg Ala Leu Arg Tyr Phe Ser Ala Ile Gln Ala His
100 105 110

Asp Gly His Trp Pro Gly Asp Phe Pro Gly Pro Leu Phe Thr Thr Ala
115 120 125

Thr Met Ile Val Val Leu Tyr Val Thr Glu Ser Leu Gly Thr Thr Leu
130 135 140

Ser Ser Glu His Arg Lys Glu Ile Cys Arg Tyr Leu Tyr Asn Arg Gln
145 150 155 160

Asn Met Asp Gly Gly Trp Gly Leu His Ala Glu Gly Glu Ser Ser Met
165 170 175

Leu Ser Ser Ala Leu Asn Tyr Ala Ala Leu Arg Leu Leu Gly Glu Gly
180 185 190

Ala Asp Asp Gly Pro Asp Met Ser Met Pro Lys Ala Arg Lys Trp Ile
195 200 205

His Asp His Gly Gly Ala Thr Met Ile Pro Ile Leu Gly Lys Val Trp
210 215 220

Leu Ser Val Leu Gly Val Ser Glu Trp Ser Gly Val Asn Pro Met Pro
225 230 235 240

Pro Glu Leu Phe Leu Leu Pro Ser Phe Val Pro Ile Gln Pro Gly Arg
245 250 255

Leu Trp Ser His Phe Arg Met Ala Phe Ile Pro Met Ser Tyr Leu Tyr
260 265 270

Gly Lys Lys Phe Val Gly Pro Ile Thr Lys Leu Val Leu Ser Leu Arg
275 280 285

Glu Glu Leu His Ile His Pro Tyr Lys Lys Ile Asn Trp Lys Gln Ala
290 295 300

Arg Lys Leu Cys Ala Lys Glu Asp Ala Tyr His Pro His Thr Trp Leu
305 310 315 320

Gln Glu Cys Leu Ser Asp Cys Leu Tyr Ser Phe Gly Glu Pro Phe Leu
325 330 335

Ala Cys Trp Pro Val Ser His Met Arg Arg Lys Ala Leu Arg Gln Ile
340 345 350

Ala Asp Phe Leu Lys Tyr Glu Asp Asp Ile Ser Arg Tyr Ile Cys Ile
355 360 365

Gly Ala Ala Gln Lys Ala Leu Ser Met Leu Cys Cys Trp Ser Glu Asn
370 375 380

Pro Ser Ser Asp Ala Phe Lys Arg His Leu Ala Arg Val Ser Asp Phe
385 390 395 400

Leu Trp Leu Gly Glu Asp Gly Met Lys Val Arg Val Cys Ala Gly Gln
405 410 415

Ser Trp Asp Val Ala Phe Ala Val Gln Ala Ile Leu Ala Cys Asp Val
420 425 430

Ala Gln Glu Phe Gly Thr Thr Leu Lys Lys Ala His His Phe Ile Lys
435 440 445

Ala Ser Gln Ile Val Ser Asn Pro Thr Gly Asp Phe Ser Arg Lys Tyr
450 455 460

Arg His Ile Ser Lys Gly Gly Trp Ala Phe Gln Val Ala Asp Gln Gly
465 470 475 480

Trp Gln Val Ser Asp Cys Thr Ala Glu Ala Leu Lys Ala Leu Leu Leu
485 490 495

Leu Ser Lys Phe Pro Ser Glu Ile Val Gly Asp Gln Met Glu Thr Cys
500 505 510

Arg Phe His Asp Ala Val Asn Ile Leu Leu Ser Leu Gln Asn Pro Asn
515 520 525

Gly Gly Tyr Gly Thr Trp Glu Leu Ala Arg Thr Tyr Pro Trp Met Glu
530 535 540

Asn Leu Asn Met Thr Glu Ile Tyr Ala Asp Ile Met Val Glu His Gln
545 550 555 560

Tyr Val Glu Cys Thr Ser Ser Val Ile Gln Ala Leu Ala Leu Phe Arg
565 570 575

Gln Lys Tyr Pro Gly His Arg Glu Asp Glu Val Glu Gln Cys Ile Arg
580 585 590

Arg Ala Thr Glu Phe Ile Glu Lys Leu Gln Asn Glu Asp Gly Ser Trp
595 600 605

Phe Gly Ser Trp Gly Ile Cys Phe Thr Tyr Gly Thr Trp Phe Ala Ile
610 615 620

Glu Gly Leu Ser Ala Val Gly Gln Cys Tyr Asn Asn Ser Thr Tyr Ile
625 630 635 640

Arg Lys Gly Cys Gln Phe Leu Leu Ser Lys Gln Leu Arg Asn Gly Gly
645 650 655

Trp Gly Glu Ser His Leu Ser Ser Thr Thr Lys Ala Tyr Thr Asn Leu
660 665 670

Asp Gly Glu Lys Ser His Val Val Asn Thr Ala Trp Ala Met Leu Ala
675 680 685

Leu Met Lys Ala Gly Gln Ala Glu Arg Asp Pro Ser Pro Leu His Glu
690 695 700

Ala Ala Arg Leu Ile Met Ser Met Gln Leu Gly Asn Gly Asp Phe Pro
705 710 715 720

Gln Glu Glu Met Ile Gly Ser Phe Leu Lys Asn Gly Pro Leu Cys Tyr
725 730 735

Met Ala Tyr Arg Asn Ile Phe Pro Ile Trp Ala Leu Gly Glu Tyr His
740 745 750

Arg Leu Val Leu Cys Ser Gly
755

<210> 16
<211> 2280
<212> DNA

<213> Barley Hordeum vulgare
<400> 16
atgtggaaac tcaagatcgc tgagggtggc ccgtggctca cgagtggcaa caatcatgcc 60
ggaagagaaa catgggagtt tgaccaaaac gacgccggat caagcgaaga ccgggatgcg 120
gtcgatgctg cacggtctga attccagaag aacaggttta ggacaaggca cagctctgat 180
gttttggctc gcctgcagct agcaaaggag aataacttca gccttgacca aaaacagaaa 240
ccaaaagatg atgaaactag tgtcgttatc aatgtagcta cggtgtcgga gacactggaa 300
agggcactcg gatacttctc ggccatacaa gcgcatgatg ggcactggcc aggagatttt 360
ccggggccac tctttaccac agcaaccatg atcatagttt tgtatgtcac ggagtcgtta 420
ggtagtacgc tatcatcaga acatcgcaag gagatctgtc gctatttgta caaccgtcag 480
aatatagatg gggatgggg actacacgcg gaaggcgaaa gctccatgct cagctcagct 540
ctcaactaca ctgctctaag actgcttggc gagggtgttg atgatggacc agacatgtcc 600
atgccgaaag caaggaaatg gatacatgac catggcggtg caacgatggt accaatcttg 660
ggaaaagtgt ggctctcggt gcttggagtt ttcgaatggt caggtgtaaa ccctattccc 720
ccagaattgt tccttctgcc atcctttgtt cctattcaac caggaagatt gtggagccac 780
ttcagaatgg ctttcattcc catgtcctat ttgtatggca agaaatttgt tggcccaata 840
accaaattag ttttgtcatt aagagaagag ctgcatattc atccctacaa aaagattaac 900
tggaggcaag cacgcaaatt atgtgcaaag gaagacgcct atcacccaca cacctggctt 960
caagaatgct tgtctgactg cctttacagt tttggcgaac cttttctggc acgttggccg 1020
gtttcctaca tgagaagaag agctctacga caaattgccg agttcctgaa atacgaagat 1080
gacaactcac ggtatatctg catcggcgcc gcgcaaaagg cactatccat gttatgctgt 1140
tggtctgaga atcccaattc agatgcattc aagaaccact tggctagagt tgctgatttc 1200
ctgtggctcg gcgaagatgg gatgaaagtt cgggtgtgtg caggtcaatc atgggatgtc 1260
gctttcgccg tgcaagcgat actagcatgt agtgttgcag aggaatttgg aagtactctc 1320
aagaaagcac atcatttcat aaaagcgtca cagattttgg acaatccttc tggtgatttc 1380
ggcagaaggt accgtcacat ttctaaagga ggatgggcct tccaggttgc agatcagggt 1440
tggcaggttt cagattgcac agcagaagct cttaaggctc tgttactgct ctcaaagttt 1500
ccgtcagata tcgttggcga tcagatggaa acatgccgct accatgatgc ggtgaatgta 1560
ttactatctt tacagaatcc taatggtggc tatgggactt gggagctagc tcgtacgtat 1620
ccatggatgg agaatttaaa catgacagag atatatgcag acatcatggt ggagcatcag 1680
tacgtcgagt gcacctcgtc ggccatccag gcattggccc tgtttcggca aaaataccccc 1740
gggcatcggg aagatgaaat agaacagtgc atcaggagag cgacagagtt catcgagaag 1800
ttacagaatg aggacggttc atggtttgga tcatggggta tttgcttcac atatggcaca 1860
tggttcgcta tagagggcct gtcagcagtt ggacagtgtt acgataacag cacctacatt 1920
cggaaggctt gccagtttct attatcgaag cagctaacga atggtggatg ggtgagagt 1980
catctttcat ccacaaccaa ggcatacacg aacctagaag gggagaaatc gcatgtagtc 2040
aacacggcgt gggcaatgtt ggcactaatg aaagctggac aggccgaaag agatccatct 2100
cctttgcacg aagcagcaag acttatcatg agcatgcagc ttggcaatgg tgacttccca 2160
caggaggaaa tgattggaag tttcttgaaa aatggcccct tgtgttatat ggcttatcgc 2220
aatatattcc ccatatgggc ccttggagag tatcataaat tagtacttta ctctgactaa 2280

<210> 17
<211> 759
<212> PRT
<213> Barley Hordeum vulgare
<400> 17

Met Trp Lys Leu Lys Ile Ala Glu Gly Gly Pro Trp Leu Thr Ser Gly
1 5 10 15

Asn Asn His Phe Gly Arg Glu Thr Trp Glu Phe Asp Arg Asn Asp Ala
20 25 30

Gly Ser Ser Glu Glu Arg Asp Ala Val Asp Ala Ala Arg Ala Glu Phe
35 40 45

Gln Lys Asn Arg Phe Arg Thr Arg His Ser Ser Asp Val Leu Ala Arg
50 55 60

Met Gln Leu Val Lys Gly Asn Asn Phe Ser Leu Asp Gln Gln Gln Lys
65 70 75 80

Pro Lys Gly Asp Glu Thr Ser Val Asp Ile Asn Ile Ala Thr Val Ser

85 90 95

Glu Thr Leu Lys Arg Ala Leu Arg Tyr Phe Ser Ala Ile Gln Ala His
100 105 110

Asp Gly His Trp Pro Gly Asp Phe Pro Gly Pro Leu Phe Thr Thr Ala
115 120 125

Thr Met Ile Val Val Leu Tyr Val Thr Glu Ser Leu Gly Thr Thr Leu
130 135 140

Ser Ser Glu His Arg Lys Glu Ile Cys Arg Tyr Leu Tyr Asn Arg Gln
145 150 155 160

Asn Met Asp Gly Gly Trp Gly Leu His Ala Glu Gly Glu Ser Ser Met
165 170 175

Leu Ser Ser Ala Leu Asn Tyr Ala Ala Leu Arg Leu Leu Gly Glu Gly
180 185 190

Ala Asp Asp Gly Pro Asp Met Ser Met Pro Lys Ala Arg Lys Trp Ile
195 200 205

His Asp His Gly Gly Ala Thr Met Ile Pro Ile Leu Gly Lys Val Trp
210 215 220

Leu Ser Val Leu Gly Val Ser Glu Trp Ser Gly Val Asn Pro Met Pro
225 230 235 240

Pro Glu Leu Phe Leu Leu Pro Ser Phe Val Pro Ile Gln Pro Gly Arg
245 250 255

Leu Trp Ser His Phe Arg Met Ala Phe Ile Pro Met Ser Tyr Leu Tyr
260 265 270

Gly Lys Lys Phe Val Gly Pro Ile Thr Lys Leu Val Leu Ser Leu Arg
275 280 285

Glu Glu Leu His Ile His Pro Tyr Lys Lys Ile Asn Trp Lys Gln Ala
290 295 300

Arg Lys Leu Cys Ala Lys Glu Asp Ala Tyr His Pro His Thr Trp Leu
305 310 315 320

Gln Glu Cys Leu Ser Asp Cys Leu Tyr Ser Phe Gly Glu Pro Phe Leu
325 330 335

Ala Cys Trp Pro Val Ser His Met Arg Arg Lys Ala Leu Arg Gln Ile
340 345 350

Ala Asp Phe Leu Lys Tyr Glu Asp Asp Ile Ser Arg Tyr Ile Cys Ile

355 360 365

Gly Ala Ala Gln Lys Ala Leu Ser Met Leu Cys Cys Trp Ser Glu Asn
370 375 380

Pro Ser Ser Asp Ala Phe Lys Arg His Leu Ala Arg Val Ser Asp Phe
385 390 395 400

Leu Trp Leu Gly Glu Asp Gly Met Lys Val Arg Val Cys Ala Gly Gln
405 410 415

Ser Trp Asp Val Ala Phe Ala Val Gln Ala Ile Leu Ala Cys Asp Val
420 425 430

Ala Gln Glu Phe Gly Thr Thr Leu Lys Lys Ala His His Phe Ile Lys
435 440 445

Ala Ser Gln Ile Val Ser Asn Pro Thr Gly Asp Phe Ser Arg Lys Tyr
450 455 460

Arg His Ile Ser Lys Gly Gly Trp Ala Phe Gln Val Ala Asp Gln Gly
465 470 475 480

Trp Gln Val Ser Asp Cys Thr Ala Glu Ala Leu Lys Ala Leu Leu Leu
485 490 495

Leu Ser Lys Phe Pro Ser Glu Ile Val Gly Asp Gln Met Glu Thr Cys
500 505 510

Arg Phe His Asp Ala Val Asn Ile Leu Leu Ser Leu Gln Asn Pro Asn
515 520 525

Gly Gly Tyr Gly Thr Trp Glu Leu Ala Arg Thr Tyr Pro Trp Met Glu
530 535 540

Asn Leu Asn Met Thr Glu Ile Tyr Ala Asp Ile Met Val Glu His Gln
545 550 555 560

Tyr Val Glu Cys Thr Ser Ser Val Ile Gln Ala Leu Ala Leu Phe Arg
565 570 575

Gln Lys Tyr Pro Gly His Arg Glu Asp Glu Val Glu Gln Cys Ile Arg
580 585 590

Arg Ala Thr Glu Phe Ile Glu Lys Leu Gln Asn Glu Asp Gly Ser Trp
595 600 605

Phe Gly Ser Trp Gly Ile Cys Phe Thr Tyr Gly Thr Trp Phe Ala Ile
610 615 620

Glu Gly Leu Ser Ala Val Gly Gln Cys Tyr Asn Asn Ser Thr Tyr Ile

625 630 635 640

Arg Lys Gly Cys Gln Phe Leu Leu Ser Lys Gln Leu Arg Asn Gly Gly
645 650 655

Trp Gly Glu Ser His Leu Ser Ser Thr Thr Lys Ala Tyr Thr Asn Leu
660 665 670

Asp Gly Glu Lys Ser His Val Val Asn Thr Ala Trp Ala Met Leu Ala
675 680 685

Leu Met Lys Ala Gly Gln Ala Glu Arg Asp Pro Ser Pro Leu His Glu
690 695 700

Ala Ala Arg Leu Ile Met Ser Met Gln Leu Gly Asn Gly Asp Phe Pro
705 710 715 720

Gln Glu Glu Met Ile Gly Ser Phe Leu Lys Asn Gly Pro Leu Cys Tyr
725 730 735

Met Ala Tyr Arg Asn Ile Phe Pro Ile Trp Ala Leu Gly Glu Tyr His
740 745 750

Arg Leu Val Leu Cys Ser Gly
755

<210> 18
<211> 735
<212> PRT
<213> Sorghum Sorghum bicolor
<400> 18

Met Trp Arg Leu Lys Val Ala Arg Gly Gly Pro Trp Leu Arg Ser Thr
1 5 10 15

Asn Gly Phe Ile Gly Arg Ala Val Trp Glu Phe Asp Pro Asp Leu Gly
20 25 30

Thr Pro Glu Glu Arg Ala Glu Val Asp Arg Val Arg Arg Glu Phe Ser
35 40 45

Asp Arg Arg Phe His Arg Arg Glu Ser Ala Asp Leu Leu Met Arg Met
50 55 60

Gln Cys Ala Lys Gln Lys Arg Tyr Gln Arg Asp Leu Pro Cys Leu Lys
65 70 75 80

Leu Glu Glu Asp Glu Asn Val Thr Glu Glu Ile Val Val Ser Ser Leu
85 90 95

Arg Arg Ala Leu Asp Gln Phe Ser Ser Leu Gln Ala Ser Asp Gly His
100 105 110

Trp Pro Gly Asp Phe Ser Gly Ile Met Phe Ile Met Pro Gly Leu Ile
115 120 125

Phe Ala Leu Tyr Val Thr Gly Ser Met Asn Val Val Ile Ser Pro Glu
130 135 140

His Arg Arg Glu Ile Cys Arg Tyr Ile Tyr Asn His Gln Asn Glu Asp
145 150 155 160

Gly Gly Trp Gly Leu His Ile Glu Gly His Ser Thr Met Leu Ser Ser
165 170 175

Ala Leu Asn Tyr Val Ala Leu Arg Leu Leu Gly Glu Cys Pro Asn Gly
180 185 190

Gly Asp Gly Ala Met Glu Lys Gly Arg Asn Trp Ile Leu Asp His Gly
195 200 205

Gly Ala Ile Phe Met Ala Ala Trp Gly Lys Phe Trp Leu Ser Val Leu
210 215 220

Gly Val Tyr Asp Trp Ser Gly Asn Asn Pro Val Pro Pro Glu Leu Trp
225 230 235 240

Leu Leu Pro His Tyr Leu Pro Phe His Pro Gly Arg Val Val Cys Tyr
245 250 255

Cys Arg Met Val Tyr Met Pro Met Ser Tyr Ile Tyr Gly Arg Arg Phe
260 265 270

Val Gly Pro Ile Thr Pro Leu Val Leu Glu Leu Arg Lys Glu Leu His
275 280 285

Thr Trp Leu Gln Glu Cys Leu Ser Asp Cys Leu Tyr Ser Phe Gly Glu
290 295 300

Pro Phe Leu Thr Arg Trp Pro Ile Ser Tyr Met Arg Lys Lys Ala Leu
305 310 315 320

Lys Gln Val Ala Glu Phe Leu Lys Tyr Glu Asp Glu Asn Ser Gln Tyr
325 330 335

Ile Cys Ile Gly Ala Ala Gln Lys Ala Leu Ser Met Leu Cys Cys Trp
340 345 350

Ile Glu Lys Ser Asn Ser Asp Ala Phe Lys Arg His Leu Ala Arg Val
355 360 365

Ala Asp Phe Leu Trp Ile Gly Glu Asp Gly Met Lys Val Arg Val Cys
370 375 380

```
Ala Gly Gln Leu Trp Asp Val Ala Phe Ala Val Gln Ala Ile Leu Ala
385                 390                 395                 400

Cys Asn Ile Ala Glu Glu Tyr Arg Ser Thr Leu Lys Arg Ala His Asp
                405                 410                 415

Phe Ile Lys Ala Ser Gln Ile Met Asp Asn Pro Ser Gly Asp Phe Ser
            420                 425                 430

Arg Lys Tyr Arg His Ile Ser Gly Gly Trp Gly Phe Gln Val Ala Asp
        435                 440                 445

Gln Gly Trp Gln Val Ser Asp Cys Thr Ala Glu Ala Leu Lys Val Leu
    450                 455                 460

Leu Met Leu Ser Lys Phe Ser Ser Asp Ile Gly Ser Asp Gln Met Glu
465                 470                 475                 480

Thr Cys Arg Leu Tyr Asn Ala Val Asn Val Leu Leu Ser Leu Gln Asn
                485                 490                 495

Pro Asn Gly Gly Tyr Gly Thr Trp Glu Leu Ala Arg Thr Tyr Pro Trp
            500                 505                 510

Met Glu Ile Phe Asp Met Thr Glu Ile Tyr Ala Asp Ile Met Val Ala
        515                 520                 525

His Gln His Val Lys Cys Thr Ser Ser Val Met Gln Ala Leu Ala Leu
    530                 535                 540

Phe Lys Glu Lys Tyr Pro Trp His Arg Lys Asp Glu Ile Asp Gln Cys
545                 550                 555                 560

Ile Arg Gly Ala Thr Glu Phe Ile Glu Lys Leu Gln Asn Asp Asp Gly
                565                 570                 575

Ser Trp Phe Gly Ser Trp Gly Ile Cys Phe Thr Tyr Gly Thr Trp Phe
            580                 585                 590

Ala Ile Glu Gly Leu Ser Ala Val Gly Gln Ser Tyr Gly Asn Ser Thr
        595                 600                 605

Cys Ile Arg Lys Ala Cys Lys Phe Leu Leu Thr Lys Gln Leu Asn Asn
    610                 615                 620

Gly Gly Trp Gly Glu Ser Tyr Leu Ser Ser Arg Thr Lys Ala Tyr Thr
625                 630                 635                 640

Asn Leu Asp Arg Gln Lys Ser His Ile Val Asn Thr Ala Trp Ala Met
                645                 650                 655
```

Leu Ala Leu Met Lys Ala Gly Gln Val Glu Arg Asp Pro Thr Pro Leu
660 665 670

His Lys Ala Ala Arg Leu Ile Met Ser Met Gln Leu Gly Asn Gly Asp
675 680 685

Phe Pro Gln Glu Glu Met Ile Gly Ser Phe Leu Lys Asn Gly Pro Leu
690 695 700

Cys Tyr Met Ala Tyr Arg Asn Ile Phe Pro Ile Trp Ala Leu Gly Glu
705 710 715 720

Tyr Gln Lys Leu Val Leu Gln Cys Asp Leu Gln Arg Pro Thr Phe
725 730 735

<210> 19
<211> 2211
<212> DNA
<213> Sorghum Sorghum bicolor
<400> 19

```
atgtggaggc tgaaggtggc gcggggcggg ccatggctgc ggtcgaccaa cggcttcatt 60
ggacgggcag tgtgggagtt cgacccggac cttggcacgc cggaggagcg cgccgaggtg 120
gacagggtcc gccgggagtt ctccgaccgc cgcttccaca ggagggagtc cgccgacctc 180
ctcatgcgca tgcagtgcgc aaagcagaag agatatcaac gtgatctgcc gtgcctcaaa 240
cttgaggagg atgagaatgt cactgaagaa attgtagtga gctccttgag gcgggctctg 300
gatcagttct cttcgctgca agcaagtgat ggtcactggc ctggtgattt cagtgggatc 360
atgttcatca tgcctggttt gatatttgcc ttgtatgtca ctggatcaat gaatgttgtc 420
atatcaccgg aacatcggcg tgagatatgt cgctatattt acaatcatca gaatgaagat 480
ggagggtggg gattgcacat cgagggccac agcaccatgc tcagctcagc cttgaactat 540
gttgctttga gattgcttgg ggagtgccca aatggcggag atggagccat ggagaaaggc 600
cgaaactgga tactggatca tggaggagcc atatttatgg cagcatgggg aaagttttgg 660
ctctcagtac tcggagtata tgattggtcc ggaaataacc cggtaccacc agaattgtgg 720
ctactaccac attacctgcc gtttcaccca ggtcgagtgg tttgctattg ccgaatggtg 780
tatatgccca tgtcttacat ttatggaagg aggttcgttg gccccataac accactagta 840
ctggaattaa gaaaagagct acatacatgg ctacaggagt gtctatctga ctgcctttac 900
agttttggtg aaccttttct cacacgttgg ccaatttcct acatgagaaa gaaagcccta 960
aaacaagtgg ctgaattctt gaaatacgaa gatgagaatt ctcaatacat ctgcattggt 1020
gccgcacaga aggcattgtc catgttgtgc tgttggatcg aaaaatccaa ctcagatgca 1080
ttcaagcgcc atttggctag agttgctgat ttcctatgga ttggtgaaga tggcatgaaa 1140
gtgcgggtct gtgctggtca actatgggat gttgcttttg cagtccaagc aatattagca 1200
tgtaatattg cagaagaata tagaagcacc ctcaagagag cgcatgattt cataaaagcc 1260
tcccagatca tggacaatcc ttctggtgac ttcagcagaa agtaccgtca catatctnaa 1320
ggagggtggg gcttccaggt tgcagatcag gggtggcagg tttcagattg cacagcagaa 1380
gctcttaagg ttttgttaat gctgtccaaa ttttcatcag acattggtag tgatcagatg 1440
gaaacatgcc gcttatataa tgcagtgaat gtattattat ccttacagaa cccaaacggt 1500
ggctatggaa cttgggagct agctcgcaca tatccatgga tggagatttt tgacatgaca 1560
gagatatatg cagatatcat ggtggcgcat cagcacgtca agtgtacctc atcagtcatg 1620
caagcattag cattgttcaa ggagaagtac ccttggcatc gaaaagatga aatagatcaa 1680
tgcattaggg gagctactga attcattgag aagttacaga atgatgatgg ctcatggttt 1740
ggatcttggg gtatttgttt cacatatggc acatggtttg ctattgaggg tctatcagct 1800
gttggacaga gttatggtaa cagcacttgt atccggaagg cctgtaagtt tctcttaaca 1860
aagcagctaa acaatggtgg atggggtgag agttatcttt catcaagaac caaggcatac 1920
acaaatctgg ataggcagaa gtcacacata gtgaacactg catgggcaat gttggcgctc 1980
atgaaagctg gacaggtgga aagagatcct actccactgc acaaagctgc aagacttatc 2040
atgagtatgc agctcggcaa tggtgacttc ccacaggagg aaatgattgg aagtttcttg 2100
aaaaatggtc ccttgtgcta catggcttat cgtaacatat ttcctatatg ggcgcttgga 2160
gagtatcaga aattagtcct ccagtgtgac ctccaacggc caaccttcta g 2211
```

<210> 20
<211> 774
<212> PRT
<213> maize Zea mays
<400> 20

Met Trp Arg Leu Lys Ile Gly Glu Gly Gly Pro Trp Leu Lys Ser Gly
1 5 10 15

```
Asn Gly His Ile Gly Arg Glu Thr Trp Glu Phe Asp Glu Asp Phe Gly
            20                  25                  30

Ser Glu Ala Asp Arg Glu Ala Val Asp Ser Ala Arg Glu Glu Phe Ser
        35                  40                  45

Lys Asn Arg Leu Arg Met Arg His Ser Ser Asp Leu Leu Ala Arg Met
    50                  55                  60

Gln Ile Ala Lys Glu Asn Gly Phe Ser Leu Asp Leu His Lys Thr Arg
65                  70                  75                  80

Asp Asp Asp Gly Arg Ser Pro Leu Leu Val Ala Thr Thr Gly Ser Ser
                85                  90                  95

Thr Val Ser Glu Thr Leu Arg Lys Ala Leu Asp Tyr Phe Ala Ala Ile
            100                 105                 110

Gln Ala His Asp Gly His Trp Pro Gly Asp Phe Pro Gly Pro Leu Phe
        115                 120                 125

Thr Thr Ala Thr Met Ile Thr Val Leu Tyr Val Thr Gly Ser Leu Asp
    130                 135                 140

Ser Thr Leu Ser Ser Glu His Arg Arg Glu Ile Cys Arg Tyr Leu His
145                 150                 155                 160

Asn Arg Gln Asn Ala Asp Gly Gly Trp Gly Leu His Ala Glu Gly Glu
                165                 170                 175

Ser Ser Met Leu Ser Thr Ala Leu Asn Tyr Thr Ala Leu Arg Leu Leu
            180                 185                 190

Gly Glu Gly Gly Asp Gly Gly Gly Gly Pro Gly Met Ser Ser Ser Ser
        195                 200                 205

Met Leu Ile Arg Ala Arg Lys Trp Ile Arg Asp Arg Gly Gly Ala Thr
    210                 215                 220

Met Ile Pro Ile Leu Gly Lys Val Trp Leu Ser Val Leu Gly Val Phe
225                 230                 235                 240

Glu Trp Ser Gly Val Asn Pro Ile Pro Pro Glu Leu Phe Leu Phe Pro
                245                 250                 255

Ser Trp Val Pro Ile Gln Pro Gly Arg Leu Trp Ser His Phe Arg Met
            260                 265                 270

Ala Phe Ile Pro Met Ser Tyr Leu Tyr Gly Lys Lys Phe Val Gly Pro
        275                 280                 285
```

```
Ile Thr Arg Leu Val Val Ser Leu Arg Glu Glu Leu His Val His Pro
    290                 295                 300

Tyr Glu Lys Ile Asp Trp Lys Ala Ala Arg Asn Ser Cys Ala Lys Glu
305                 310                 315                 320

Asp Val Tyr Ser Pro His Thr Trp Leu Gln Glu Cys Leu Ser His Cys
                325                 330                 335

Leu Tyr Ser Phe Gly Glu Pro Phe Leu Thr Arg Trp Pro Ile Ser Tyr
            340                 345                 350

Met Arg Lys Lys Ala Leu Gln Gln Val Ala Glu Phe Leu Lys Tyr Glu
        355                 360                 365

Asp Glu Asn Ser Gln Tyr Ile Cys Ile Gly Ala Ala Gln Lys Ala Leu
    370                 375                 380

Ser Met Leu Cys Cys Trp Ile Glu Lys Ser Asn Ser Asp Ala Phe Lys
385                 390                 395                 400

Arg His Leu Ala Arg Val Ala Asp Phe Leu Trp Ile Gly Glu Asp Gly
                405                 410                 415

Met Lys Val Arg Val Cys Ala Gly Gln Leu Trp Asp Val Ala Phe Ala
            420                 425                 430

Val Gln Ala Ile Leu Ala Cys Asn Ile Ala Glu Glu Tyr Arg Gly Thr
        435                 440                 445

Leu Lys Lys Ala His Asp Phe Ile Lys Ala Ser Gln Ile Met Asp Asn
    450                 455                 460

Pro Ser Gly Asp Phe Ser Arg Lys Tyr Arg His Ile Ser Lys Gly Gly
465                 470                 475                 480

Trp Gly Phe Gln Val Ala Asp Gln Gly Trp Gln Val Ser Asp Cys Thr
                485                 490                 495

Ala Glu Ala Leu Lys Val Leu Leu Met Leu Ser Arg Phe Ser Ser Asp
            500                 505                 510

Ile Gly Ser Asp Gln Met Glu Thr Cys Arg Leu Tyr Asp Ala Val Asn
        515                 520                 525

Val Leu Leu Ser Leu Gln Asn Pro Asn Gly Gly Tyr Gly Thr Trp Glu
    530                 535                 540

Leu Ala Arg Thr Tyr Pro Trp Ile Glu Ile Phe Asn Met Thr Glu Ile
545                 550                 555                 560
```

```
Tyr Ala Asp Ile Met Val Glu His Gln Tyr Val Glu Cys Thr Ser Ser
                565                 570                 575

Val Met Gln Ala Leu Val Leu Phe Arg Glu Lys Asp Pro Trp His Arg
            580                 585                 590

Lys Asp Glu Ile Asp Gln Cys Ile Arg Gly Ala Thr Glu Phe Ile Glu
        595                 600                 605

Lys Leu Gln Asn Asp Asp Gly Ser Trp Phe Gly Ser Trp Gly Ile Cys
    610                 615                 620

Phe Thr Tyr Gly Thr Trp Phe Ala Ile Glu Gly Leu Ser Ala Val Gly
625                 630                 635                 640

Gln Ser Tyr Gly Asn Ser Thr Cys Ile Gln Lys Ala Cys Lys Phe Leu
                645                 650                 655

Leu Ala Lys Gln Leu Lys Asn Gly Gly Trp Gly Glu Ser His Leu Ser
            660                 665                 670

Ser Thr Thr Lys Ala Tyr Thr Asn Leu Asp Lys Glu Lys Ser His Ile
        675                 680                 685

Val Asn Thr Ala Trp Ala Met Leu Ala Leu Met Lys Ala Gly Gln Ala
    690                 695                 700

Glu Arg Asp Pro Thr Pro Leu His Lys Ala Ala Arg Leu Ile Met Ser
705                 710                 715                 720

Met Gln Leu Ser Asn Gly Asp Phe Pro Gln Glu Glu Met Ile Gly Ser
                725                 730                 735

Phe Leu Lys Asn Gly Pro Leu Cys Tyr Met Ala Tyr Arg Asn Ile Phe
            740                 745                 750

Pro Ile Trp Ala Leu Gly Glu Tyr Gln Lys Leu Val Leu Gln Cys Asp
        755                 760                 765

Leu Gln Trp Pro Thr Phe
    770
```

<210> 21
<211> 2325
<212> DNA
<213> maize Zea mays
<400> 21

```
atgtggcggc tgaagatcgg cgagggcggg ccgtggctca agagcggcaa cgggcacatt    60
ggaagggaga cgtgggagtt cgacgaggat ttcggatcgg aagcagacag ggaggccgtc   120
gactccgcgc gggaggagtt cagcaagaac cggctccgga tgcgacacag ctccgatctc   180
ttggcccgca tgcagatagc gaaggagaac ggtttcagcc tcgacctgca caagacgaga   240
gacgacgacg ggcggtctcc tctgctggtg gcgacgacag gctccagcac ggtgtcggag   300
acgctgcgaa aggcgctgga ctacttcgcg gcgatacagg cgcacgacgg gcactggcca   360
```

```
ggagacttcc cagggccgct gttcaccaca gcaaccatga tcacggttct gtacgtgacg    420
gggtcgttgg acagcacgct gtcgtcggag caccgcaggg agatctgccg ctacctgcac    480
aaccgccaga acgcggacgg ggggtggggg ctgcacgcgg aaggcgaaag ctccatgctc    540
agcacagctc tcaactacac cgctctcagg ctgctcggcg agggcggaga cggcggcggc    600
gggcccggca tgtcgtcgtc gtccatgctt attagagcga ggaagtggat acgtgatcgt    660
ggtggcgcca ccatgatacc catccttggg aaagtgtggc tctcggtgct tggagttttc    720
gaatggtcag gcgtaaaccc catcccacca gaactgtttc ttttcccatc ctgggttcct    780
attcaaccag gacggctgtg gagccacttc aggatggcgt tcattcccat gtcctatttg    840
tacggcaaga aatttgtcgg gcccatcacc agattggtcg tgtcgctacg ggaagagctg    900
catgtccacc cctacgaaaa gattgactgg aaggcagctc gtaattcatg tgccaaggaa    960
gatgtgtata gtccacatac gtggctgcag gagtgtctat ctcactgcct ttacagcttc   1020
ggcgaacctt ttctcacccg ttggccaatt tcctacatga gaaagaaagc cctacagcaa   1080
gtcgctgaat tcttgaaata cgaagacgag aattctcaat acatctgcat tggtgctgca   1140
cagaaggcac tgtccatgtt gtgctgctgg atcgaaaaat ccaactcaga tgcgttcaag   1200
cgacatttgg ccagagtcgc tgatttccta tggatcggtg aagatggcat gaaagtgcgg   1260
gtctgtgctg gtcagctatg ggatgttgct ttcgcagtcc aagcaatact agcatgtaac   1320
atcgcagaag aatacagagg caccctcaag aaagcgcacg atttcataaa ggcctcccag   1380
atcatggaca atccttctgg tgacttcagc agaaagtacc gccacatatc taagggaggg   1440
tggggcttcc aggttgcaga tcaggggtgg caggtttcag attgcacagc agaagctctc   1500
aaggttttgt taatgctgtc cagattttca tcagacattg gaagcgatca gatggaaaca   1560
tgccgcctat atgatgcagt gaatgtgttg ttatccttac agaacccaaa cggtggctat   1620
ggaacttggg agctagctcg cacatatcca tggatagaga ttttcaacat gacagagata   1680
tacgcagaca tcatggtgga gcatcagtac gtcgagtgta cctcatcagt catgcaagca   1740
ttagtattgt ttcgggagaa ggacccttgg catcgaaaag atgaaataga ccaatgcatt   1800
aggggagcta ctgaattcat cgagaagttg cagaatgatg atggctcatg gtttggatct   1860
tggggtattt gtttcacata tggcacatgg tttgctattg agggtctatc agctgttggg   1920
cagagttatg gtaacagcac ttgtatccag aaggcctgca agtttctctt agcaaagcag   1980
ctaaagaatg gtggatgggg tgagagtcat ctttcatcta caaccaaggc atacacaaat   2040
ctggataagg agaagtcaca catagtgaac actgcgtggg caatgttggc gctcatgaaa   2100
gctggacagg cggaaagaga tcccactcca cttcacaaag ctgcacggct tatcatgagt   2160
atgcagctca gcaatggcga cttcccacag gaggaaatga tcggaagttt cttgaaaaat   2220
ggccccttgt gttacatggc ttatcgcaac atatttccta tatgggcgct tggagagtat   2280
cagaaattag tcctccagtg tgacctccaa tggccaacct tctag                   2325
```